# EUROPEAN PATENT SPECIFICATION

(11) **EP 4 158 071 B1**
(45) Date of publication and mention of the grant of the patent: **29.07.2026**
(21) Application number: 21732975.4
(22) Date of filing: 18.05.2021
(51) Int. Cl.: C12Q 1/689, C12Q 1/6825

(54) **PROBE FOR THE DETECTION OF BACTERIAL INFECTIONS**
SONDE ZUM NACHWEIS BAKTERIELLER INFEKTIONEN
SONDE POUR LA DÉTECTION D'INFECTIONS BACTÉRIENNES

(30) Priority: 27.05.2020 IT 202000012496
(43) Date of publication of application: 05.04.2023
(73) Proprietor: Università Degli Studi di Udine, 33100 Udine (IT); Institut national de recherche pour l'agriculture, l'alimentation et l'environnement, 75007 Paris (FR)
(72) Inventor: VIZZINI, Priya, 33100 Udine (IT); MANZANO, Marisa, 33100 Udine (IT); VIDIC, Jasmina, 75007 Paris (FR); RAMA RAO, Nalini, 75007 Paris (FR)
(74) Representative: Predazzi, Valentina
(86) International application number: PCT/IB2021/054241
(87) International publication number: WO 2021/240299

(56) References cited:
- WO-A1-2010/057069
- WO-A1-2010/057069
- JP-A- 2010 022 336
- JP-A- 2010 022 336
- KR-A- 20080 028 633
- KR-A- 20080 028 633
- DATABASE EMBL [online] 4 November 2019 (2019-11-04), "Campylobacter fetus strain 21B43_Ps51 16S ribosomal RNA gene, partial sequence.", XP002801891, retrieved from EBI accession no. EM_STD:MN614393 Database accession no. MN614393
- DATABASE EMBL [online] 4 November 2019 (2019-11-04), "Campylobacter fetus strain 21B43_Ps51 16S ribosomal RNA gene, partial sequence.", XP002801891, retrieved from EBI accession no. EM_STD:MN614393 Database accession no. MN614393

## Description

The present invention relates to a nucleotide probe specific for *Campylobacter jejuni, Campylobacter coli, Campylobacter lari* and *Campylobacter upsaliensis,* its uses, and methods for detecting said microorganisms in a sample of interest, and kits comprising said probe and suitable reagents.

### PRIOR ART

Zoonoses are infections or diseases that can be transmitted directly or indirectly between animals and humans.

Food-borne zoonoses are caused, for example, by eating contaminated food or by coming into contact with infected animals.

Food-borne diseases pose a significant and widespread threat to public health, particularly for very young and elderly people, as well as pregnant women and people susceptible to a weakened immune system.

Some 246,571 human cases of food-borne illness are confirmed at a rate of 64.1 per 100,000 inhabitants in the European Union. EFSA (2019) presents the report of confirmed human cases of 14 zoonoses, most of which are caused by *Campylobacter* spp., *Salmonella, Yersinia, Listeria monocytogenes, Escherichia coli* and *Brucella.* Campylobacteriosis has been the most common since 2005, with more confirmed and hospitalized cases.

In addition, outbreaks generate health costs amounting to millions of euros, public health problems and losses of agricultural products. In Europe, the public health cost caused by this disease alone is 2.4 billion euros per year (EFSA, 2014).

Human pathogens are commonly found in the intestines of healthy animals used for food production. Contamination risks are present from farm to table and require prevention and control during food testing. Public health authorities have established strict measures and regulations for food control systems, such as the Hazard Analysis Critical Control Point (HACCP) system to prevent the spread of these food-borne pathogens at the food processing level. HACCP is a method of ensuring food safety based on the application of good hygiene practices and identifies the necessary control measures in food operations and hygiene practices. Therefore, the detection of food borne pathogenic bacteria is a crucial parameter for the prevention and control of certain hazards in food processing.

*Campylobacter is a* Gram-negative bacterium belonging to the *Campylobacteraceae* family, which is well known worldwide as a pathogen causing gastroenteritis in humans.

*Campylobacter* is a pathogen that can proliferate both in animals, especially poultry and pigs, and in humans, albeit with similar but not identical mechanisms of invasion. *Campylobacter jejuni* and *Campylobacter coli* found in the gastrointestinal tract of different animal species account for about 90% of all bacterial *Campylobacter* diagnosed in humans in the EU. In addition to these species, *C. upsaliensis, C. lari* and *C. fetus* are also responsible for this pathogenicity. In some sporadic cases, *Campylobacter* can also be the precursor of serious diseases, such as Reiter's syndrome, irritable bowel syndrome, Guillain-Barré syndrome and Miller-Fisher syndrome, a form of chronic and potentially fatal paralysis.

European Commission Regulation 2073/2005, which specifies microbiological criteria for specific food categories, was amended by Commission Regulation (EU) 2017/1495, which added chicken carcasses as a new food category and introduced a hygiene criteria process for *Campylobacter.* The criteria for *Campylobacter* spp. are set as ≤1,000 CFU / g in the chicken carcass after cooling.

By contrast, EU Regulation 2073/2005 for foodstuffs on the market establishes microbial criteria for the pathogen to be absent in 25 g of sample.

Since 2005, *Campylobacter* spp. has continued to be the most commonly reported gastrointestinal disease bacterium in humans in the European Union, with a number of human campylobacteriosis cases of 246,517 (EFSA, 2019). Despite the high number of human campylobacteriosis cases, mortality was low (0.03%) in the reported cases. Between 2013 and 2017 there was a clear seasonality in the number of confirmed cases of campylobacteriosis reported, with a peak in the summer months. The EFSA 2019 report showed that for 55% of confirmed cases of campylobacteriosis in the EU, the following species were attributed: 83.9% *C. jejuni,* 10.3% *C*. *coli,* 0.1% *C. lari,* 0.1% *C. upsaliensis* and 0.1% *C. fetus.*

Campylobacteriosis occurs after eating contaminated food. The lowest source is milk and dairy products, including cheese, with an incidence of less than 0.6%. By contrast, the occurrence of *Campylobacter* in fresh meat is high, 37.5% for chicken, 28.2% for turkey meat 23.8% for other poultry meat, 5.8% for pork and 0.5% for beef.

The standard method ISO 10272-1: 2006 for detecting *Campylobacter* spp. which was recently replaced by ISO 10272-1: 2017, was not optimal for detecting the bacterium in food matrices with a rich contaminating microbial flora. Therefore, the following main changes were made: the detection method was extended to include the option of a second enrichment broth (Preston broth); the choice of an appropriate enrichment broth is an important step. Bolton Broth (BB) is able to revitalize *Campylobacter* spp. compared to Preston broth. Recently, studies have been conducted to improve the selectivity of the enrichment broth.

WO2010/057069 discloses probes specific for the detection of campylobacter species *C. Jeuni, C. lari,* and *C*. *coli* that do not bind to *C*. *fetus* and *C*. *upsaliensis.*

New methods and means for detecting *Campylobacter* spp., especially those that allow specific detection of the species of greatest health concern, are of particular interest to the food industry and to public health.

### SUMMARY OF THE INVENTION

The main objective of this invention is to provide new tools for the analysis and detection of *Campylobacter* spp. to allow rapid and sensitive diagnosis of its presence in food, or in human or animal biological samples, or even in environmental samples. The present invention provides means and methods for a rapid response analysis at low cost, the development of new investigative tools to replace those currently available allowing a reduction in the time and complexity of laboratory instrumentation and procedures, also allowing a miniaturization of the devices required for said analysis, with greater ease of use and portability for the development of a point of care (POC).

Indeed, LOCs (labs on chips) are of great interest in the agri-food sector and in environmental monitoring, for example to monitor food safety along the food chain and in environmental monitoring to prevent environmental contamination.

The present invention provides a nucleotide probe specific for *Camplylobacter jejuni, C. coli, C. upsaliensis* and *C. lari,* which allows these microorganisms to be identified in samples of interest in a rapid and specific manner.

The present invention, by means of the probe of SEQ ID NO 1, provides a more practical alternative to the classical method of *Campylobacter* isolation carried out by the official ISO:10272- 2017 method by making it possible to carry out a direct hybridization of the probe without the need to resort to amplification, and provides results with high specificity, making it possible to reduce the analysis time required to obtain the result.

The following are therefore the subject of the invention:
a nucleotide probe of SEQ ID NO 1;
a method for detecting one or more of *C. jejuni, C. coli, C. lari* and *C. upsaliensis* in a sample comprising:
   a step of DNA extracting from said sample
   a step of hybridizing the DNA extracted from said sample with the nucleotide probe as defined in any one of claims 1 to 3, and
   a step of detecting the hybridization of said probe to said DNA, in which
   the detection of hybridization of said probe to said DNA indicates the presence of at least one of *C. jejuni, C. coli, C. lari* and *C. upsaliensis* in said sample;
   a biosensor electrode on which oligonucleotide probes of SEQ ID NO 1 are immobilized;
   a biosensor comprising the electrode;
   a kit comprising one or more aliquots of oligonucleotide probe of SEQ ID NO 1 and any other appropriate reagents;

### GLOSSARY

Hybridization in this description and in the prior art means a technique that utilizes the ability of single-stranded nucleic acids that are partially or totally complementary to each other to form double-stranded molecules by pairing complementary bases. A hybridization assay is an experiment in which a well-characterized population of short single-stranded nucleic acid sequences, or oligonucleotides (referred to as probes), are used to search for target (complementary) sequences in a population of nucleic acid sequences used as targets with which to form a double-stranded molecule.

The complementary nature of a base as described here and in the prior art defines the degree to which the sequences of two single-stranded nucleic acid molecules are able to form double-stranded molecules by compulsory pairing of the nitrogenous bases described by Watson-Crick rules (A paired with T or U; C paired with G).

*In situ* hybridization according to this description and in the prior art defines a hybridization reaction in which a labelled oligonucleotide (probe) binds a nucleotide of the probe to a molecule allowing the detection of target nucleic acid molecules in a sample of interest, thus allowing the detection of the presence or absence of said target molecules, which are complementary to said probe, in the sample analyzed.

Microarray defines a solid surface on which molecules of interest can be immobilized, according to specific coordinates, in a high-density grid format for use in particular analyses. An oligonucleotide or DNA microarray consists of numerous DNA or oligonucleotide molecules located at predefined positions on the array, at regular intervals, to serve as probes in a hybridization assay. Each specific position contains several thousand identical copies of a particular oligonucleotide or DNA.

The hybridization stringency according to the present invention and in the literature is the degree to which the experimental hybridization conditions tolerate the presence of unpaired bases in the heteroduplex molecule. High-stringency conditions are defined as conditions under which only 100% complementary sequences can pair up. Low stringency conditions are defined as those conditions in which heteroduplexes can form between nucleic acid strands, even with a significant number of unpaired bases. Stringency is conventionally alleviated by lowering the pairing temperature and/or increasing the salt concentration.

Melting temperature (Tₘ). Temperature at which 50% of the heteroduplex molecule is present in the double-stranded form and 50% in the single-stranded form.

The term functionalization in the present invention has the meaning commonly used in chemistry, and indicates the incorporation or binding of a material, such as nanoparticles or electrodes, with one or more functional groups represented, in the case of the present invention, by oligonucleotides or probes of SEQ ID NO 1,
wherein said oligonucleotides or probes may in turn be labelled as described in the present description.

CampyP3 in the present description means a single-stranded DNA probe or oligonucleotide (ss DNA) of SEQ ID NO 1.

CCP3 in the present description means a DNA probe or oligonucleotide having a sequence complementary to SEQ ID NO 1.

### ABBREVIATIONS

| | |
|---|---|
| Ab | Antibody |
| ALP | Alkaline phosphatase |
| ATCC | American Type Culture Collection |
| BHI | Brain Heart Infusion |
| -Bio | Referring to Biotin-labelled oligonucleotides or probes |
| BLAST | Basic Local Alignment Search Tool |
| CampyP3 | Oligonucleotide having SEQ ID NO 1 |
| CBA | Columbia Blood Agar |
| CCD | Charge-coupled device |
| CCP3 | Oligonucleotide with a sequence complementary to SEQ ID NO 1 |
| CE | Counter electrode |
| DI4A | Department of Agri-Food, Environmental and Animal Sciences. |
| -Dig | Referring to digoxigenin-labelled oligonucleotides or probes |
| DISTAM | Department of Food and Microbiological Sciences and Technologies |
| DPV | Differential pulse voltammetry |
| DSM | Deutsche Sammlung von Mikroorganism und Zellkulturen GmbH (German Collection of Microorganisms and Cell Cultures GmbH) |
| EDTA | Ethylenediaminetetraacetic acid |
| FISH | Fluorescence in situ hybridization |
| HRP | Horseradish peroxidase |
| ICS | Isolated from clinical samples |
| Ipa | Anodic peak intensity |
| ISO | International Organization for Standardization |
| LSPR | Localized surface plasmon resonance |
| M | Molar |
| mCCDA | Modified charcoal cefoperazone deoxycholate agar |
| MCH | 6-mercapto-1-hexanol |
| mM | millimolar |
| NaAC | Sodium acetate |
| NH2 | Amine group |
| NPs | Nanoparticles |
| OLED | Organic light-emitting diode |
| PBS | Phosphate buffered saline |
| PCR | Polymerase chain reaction |
| QCM | Quartz Crystal Microbalance |
| RE | Reference electrode |
| rpm | Revolutions per minute |
| SDS | Sodium dodecyl sulphate |
| SERS | Surface-enhanced Raman spectroscopy |
| SH | Thiol group |
| SPAuEs | Screen Printed Gold Electrodes |
| SPR | Surface Plasmon Resonance |
| SSC | Sodium citrate |
| SSHP | Streptavidin Stabilized with Horseradish Peroxidase |
| TE | Tris-EDTA |
| TECP | tris(2-chloroethyl) phosphate |
| TMB | 3, 3', 5, 5' - tetramethylbenzidine |
| UV | Ultraviolet |
| V | Voltage |
| WE | Working electrode |
| XLD | Xylose Lysine Desoxycholate |

### DETAILED DESCRIPTION OF THE FIGURES

Figure 1 Sequence alignment of *Campylobacter spp.* using Multalin software.

Figure 2 Specificity results of the probe having SEQ ID NO 1 dot blot enzyme immunoassay and chemiluminescent results. Membranes *a* and *b* show the data obtained with the CampyP3-Dig probe (DNA having SEQ ID NO 1 labelled with digoxigenin) applied to the immunoenzymatic system. Membranes *c*, *d* and *e* show the data obtained with the CampyP3-Bio probe applied to the chemiluminescent system.

Membrane *a* shows the sensitivity test data. In line A and B the CCP3 probe is deposited at scalar concentrations (Line A1:100 ng / µL, A2: 50 ng / µL, A3: 10 ng / µL, A4: 1 ng / µL. CCP3 in Line B, B1: 0.1 ng / µL B2: 0.01 ng / µL B3: 0.001 ng / µL B4: 0.0001 ng / µL). At position A4 the last visible spot is present. This indicates that the detection limit is 1 ng/µL.

Membrane *b* shows the specificity test data. Line A contains the positive control (CCP3) and several negative controls such as *E. coli, S. enterica, H. pylori, Arc. butzleri.* Line B contains the positive controls, C. *jejuni, C. coli, C. lari* and *C.upsaliensis, standardized* to 100 ng/uL . By contrast, line C shows C. *jejuni, C. coli,* C. *lari* and *C.upsaliensis* at the extraction concentration. Membrane *c* shows the result of the sensitivity test. In line A and B the CCP3 probe is deposited at scalar concentrations (Line A, A1: CCP3 1 ng / µL A2: CCP3 0.5 ng / µL A3: CCP3 0.2 ng / µL A4: CCP3 0.1 ng / µL. Line B, B1: CCP3 0.05 ng / µL, B2: CCP3 0.02 ng / µL, B3: CCP3 0.01 ng / µL, B4: CCP3 0.005 ng / µL). At position A4, the last visible spot is present. This indicates that the detection limit with this system is 0.1 ng/µL.

In membrane *d,* the sensitivity test was also conducted with genomic DNA from *C. jejuni.* Different scalar concentrations of DNA were deposited in the line A. The detection limit for genomic DNA is 5 ng/µL.

Membrane *e* shows the specificity test data. Equal scalar concentrations were deposited in lines A, B and C for the CCP3, PR (SEQ ID NO 4) and PE (SEQ ID NO 5) probes, respectively. The CCP3 probe confirmed the data obtained in membrane *c.* Probes PR and PE acting as negative controls, did not give positive results.

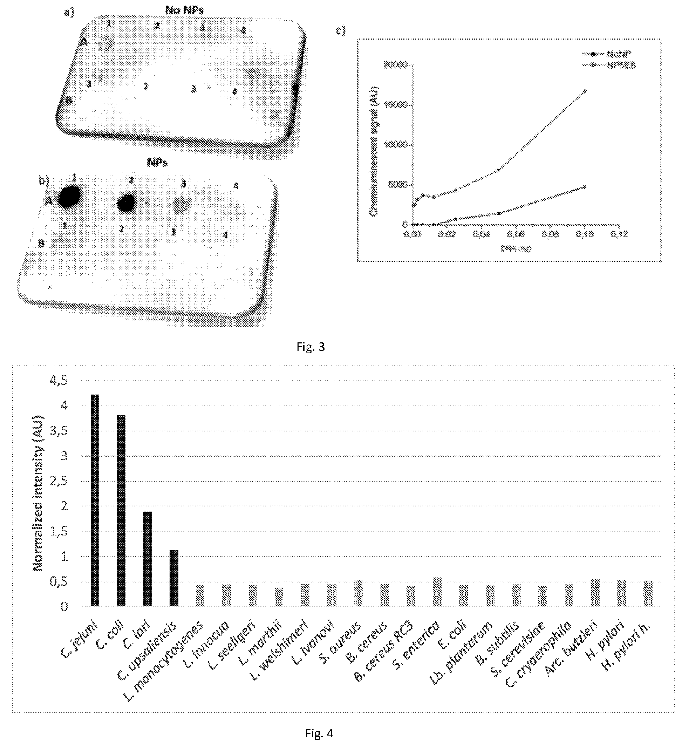

Figure 3 Membrane a shows the data of the sensitivity test with the chemiluminescence method, membrane *b* shows the data of the sensitivity test with the system based on chemiluminescence-SiNPs. Graph *c* compares the signal intensity obtained between the two methods.

Figure 4 Shown are the results of the specificity test in which the DNAs of different micro-organisms were tested. Signal intensity measurements were repeated three times. The averages of the values are shown in the following graph. The graph shows the difference in signal intensity between the positive controls in dark grey and the negative samples in light grey.

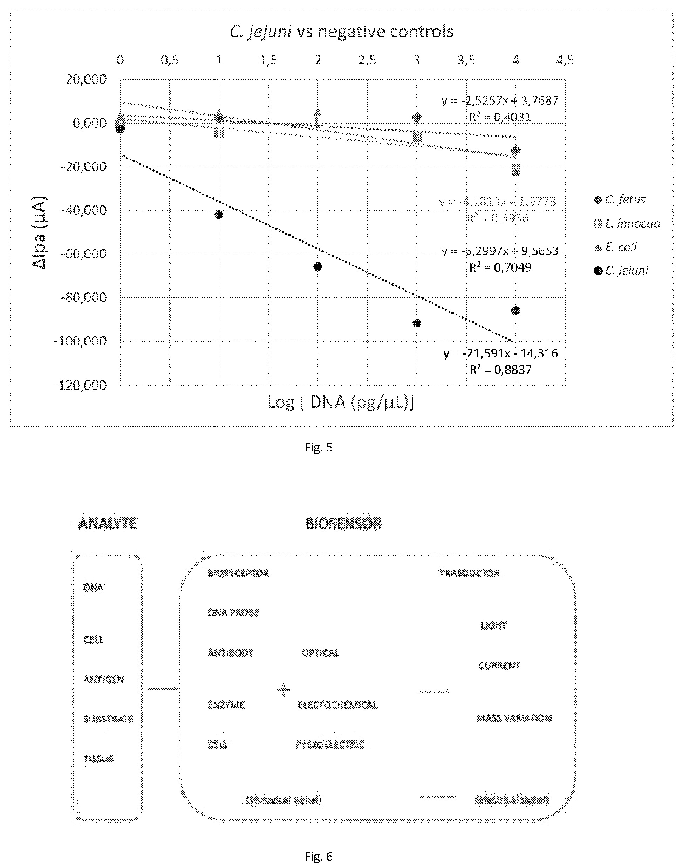

Figure 5 Calibration curves obtained by measuring the DPV of *C. fetus* (diamond), *L. innocua* (square), *E. coli* (triangle) and *C. jejuni* (circle).

Figure 6 Composition of a biosensor.

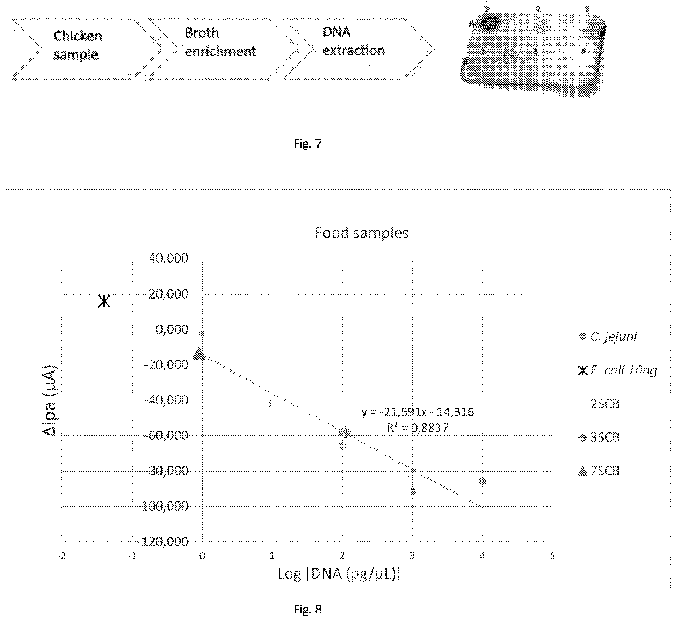

Figure 7 Results obtained by hybridization of the probe having SEQ ID NO 1 to DNA extracted from chicken samples. A schematic of the analysis of the chicken samples is shown in the figure. The chicken sample was treated with the enrichment step, DNA was extracted and analyzed by the SiNPs protocol. In *a)* the results obtained are shown; row A: A1: CCP3 0.1 ng/µL, A2: 3SCB, A3: 10SCB; in row B, B1: 4SCB, B2: 5SCB B3: 6SCB.

Figure 8 Food samples plotted in the regression line of *C. jejuni* obtained from DPV: plotted on the ordinate is the delta of the current intensity of the anodic peak, on the abscissa the DNA concentration expressed in logarithm.

### LIST OF SEQUENCES

SEQ ID NO 1
   TAGTGGCGCACGGGTGAGTAAGGTATAGTTAATCTG
SEQ ID NO 2 universal forward primer P1V1
   GCGGCGTGCCTAATACATGC
SEQ ID NO 3 universal reverse primer 518R
   ATTACCGCGGCTG
SEQ ID NO 4
   PR **CGTGCGCCACTACAGATTACCAACTATCTCACC**

The following probe represents the division of SEQ ID NO 1 into 6 parts, with each part consisting of 6 bases. The 6 parts of SEQ ID NO 1 were randomly mixed to generate the PR probe.
SEQ ID NO 5 PE AAGACGCGCATCTCTTCACCAGCGCGCTTC

The PE probe represents the sequence of highest hybridization probability of the SEQ ID NO 1 probe on the 16S gene of *E. coli* where 21 out of 36 bases match.

### DETAILED DESCRIPTION

The present description relates to a nucleotide sequence defined in SEQ ID NO 1, and in particular to a nucleotide probe, or an oligonucleotide, having SEQ ID NO 1.

A particular characteristic of this probe or oligonucleotide is that it is 100% complementary to the nucleotides in position 69-104 of the gene for 16S rRNA of *C. jejuni* GenBank access number: NR_117760.1, to nucleotides in position 71-106 of the gene for 16S rRNA of *C. coli* GenBank access number: JX912505.1, to nucleotides in position 67-102 of the gene for 16S rRNA of *C. lari* GenBank access number: NR_115289.1, and to nucleotides in position 55-90 of the gene for 16S rRNA of *C. upsaliensis* GenBank access number: NR_115289.1.

This particular characteristic allows the use of such a probe or oligonucleotide in the specific detection of microorganisms belonging to one or more of the species *Campylobacter jejuni, Campylobacter coli, Campylobacter lari* and *Campylobacter upsaliensis* in samples in which the presence of these microorganisms is to be verified, for example for sanitary reasons.

The state of this probe or oligonucleotide being 100% complementary to regions of the 16S rRNA gene in the microorganisms listed above allows the specific detection of one or more of these four species in the samples tested. The probe or oligonucleotide having SEQ ID NO 1 has the characteristics shown below in Table 1. All values reported are within the optimal parameters specified by the software.

**Table 1. Characteristics of the nucleic probe (Amplifix).**

| | |
|---|---|
| Tm | 64 |
| GC % | 47 |
| 3'-stability | 2 |
| poly x | 0 |
| self dimer | 16 |
| self end dimer | 0 |

in which,
- Tm is the melting temperature
- % GC represents the percentage in G, guanine, and C, cytosine,
- 3'-stability: indicates the stability of binding to a complementary probe at the 3' end and depends on the nucleotides of which the sequence at the 3' end is composed.
- poly x: is an indicator for the formation of repeated tandem sequences that may lead to non-specific hybridization. The value should be as close to 0 as possible.
- self dimer indicates the possibility of hybridization of the probe with itself
- self end dimer indicates the possibility of forming hairpin structures.

Probe specificity was assessed *in silico* using Amplifix, Fast PCR and BLAST.

Amplifix and FAST PCR are publicly available programs that make it possible to simulate the hybridization of oligonucleotide probes against a complementary nucleotide probe.

Amplifix found positivity for sequences corresponding to bacteria with the following access numbers (Tab. 2):

**Table 2. List of microorganisms positive for hybridization with a DNA probe having SEQ ID NO 1.**

| **N.** | **Bacteria** | **Access number** |
|---|---|---|
| **1** | *Campylobacter jejuni* | nr_117760.1 |
| **2** | *C. jejuni* | JX912519.1 |
| **3** | *C. coli* | JX912505.1 |
| **4** | *C. lari* | NR_117762.1 |
| **5** | *C. upsaliensis* | NR_115289.1 |

Amplifix found no hybridization for sequences corresponding to bacteria with the following access numbers (Tab. 3):

**Table 3. List of microorganisms negative for hybridization with a DNA probe having SEQ ID NO.**

| **N.** | **Bacteria** | **Access number** |
|---|---|---|
| **1** | *Campylobacter fetus* | nr_118514.1 |
| **2** | *C. concisus* | jx912506.1 |
| **3** | *C. gracilis* | jx912515.1 |
| **4** | *C. ureolyticus* | nr_117766.1 |
| **5** | *Escherichia coli* | nz_cp019944.1 |
| **6** | *Escherichia coli* | ef527445.1 |
| **7** | *Salmonella choleraesuis* | eu014681.1 |
| **8** | *S. enterica subsp. indica* | nr_044370.1 |
| **9** | *S. enterica subsp. salamae* | nr_044372.1 |
| **10** | *S. bonqori* | nr_116124.1 |
| **11** | *S. enterica subsp. diarizonae* | nr_044373.1 |
| **12** | *S. enterica subsp. arizonae* | nr_116125.1 |
| **13** | *S. enterica strain 365* | jq694170.1 |
| **14** | *Escherichia coli* | ef527445.1 |
| **15** | *Enterobacter cloacae subsp. dissolvens* | eu078570.1 |
| **16** | *Gallus gallus* | ab489247.1 |
| **17** | *Helicobacter ganmani* | ay277975.1 |
| **18** | *Morganella morganii* | ab089244.1 |
| **19** | *Pediococcus pentosaceus* | af268968.1 |
| **20** | *Pseudomonas fluorescens* | ef198908.1 |
| **21** | *P. fluorescens* | kx186944.1 |
| **22** | *P. migulae* | kf857261.1 |
| **23** | *Weissella cibaria* | fi429988.1 |
| **24** | *Helicobacter pylori* | ay593991.1 |
| **25** | *H. pylori* | nr_114587.1 |
| **26** | *H. pullorum* | nr_043053.1 |
| **27** | *Arcobacter suis* | FJ573216.1 |
| **28** | *A. butzleri* | NR_043035.1 |
| **29** | *A. cryaerophilus* | U25805.1 |
| **30** | *A. skirrowii* | DQ464344.1 |

The results obtained were also confirmed with the Fast PCR6.1 program.

The BLAST program (Basic Local Alignment Search Tool) is an alignment software program for comparing a sequence of interest (in this case the CampyP3 probe) with a database of known sequences and for identifying homologous sequences. This procedure is carried out to assess whether the probe sequence is identifiable only for the genus and/or species of interest. The program verified that the sequence is specific to the *Campylobacter* genus.

Analyses based on the dot blot technique, using a colorimetric and chemiluminescence detection method, and analyses based on voltammetry confirmed the specificity of the probe as described above. The tests were performed on the strains listed in Table 4.

***Campylobacter* spp. strains and other strains from international collections used for specificity testing.**
^{A}DSM: Deutsche Sammlung von Mikroorganism und Zellkulturen GmbH (German Collection of Microorganisms and Cell Cultures GmbH) (Braunschweigh, Germany).
^{B}ICS: Isolated from Clinical samples (Hospital of Udine, Italy).
^{C}ATCC: American Type Culture Collection (Manassas, VA, USA).
^{D} DI4A: Department of Agri-Food, Environmental and Animal Sciences (Udine, Italy).
^{E} DISTAM: Department of Food and Microbiological Sciences and Technologies (Milan, Italy).

| **Controls** | **N.** | **Microorganisms** | **Collection codes** | **T°C** | **Incubation (h)** |
|---|---|---|---|---|---|
| Positive | 1 | *Campylobacter jejuni subsp. jejuni* | DSM 4688 ^{A} | 37 | 48 |
| | 2 | *C. coli* | DSM 24155 | 37 | 48 |
| | 3 | *C. lari subsp.lari* | DSM 11375 | 37 | 48 |
| | 4 | *C. upsaliensis* | DSM5365 | 37 | 48 |
| Negative | 5 | *C. fetus* | DSM 5361 | 37 | 48 |
| | 6 | *C. cryaerophila* | DSM 7289 | 37 | 48 |
| | 7 | *Helicobacter pylori ¹* | DSM 7492 | 37 | 48 |
| | 8 | *H. pylori ²* | ICSS ^{B} | 37 | 48 |
| | 9 | *H. suis* | DSM 19735 | 37 | 48 |
| | 10 | *Arcobacter butzleri* | DSM 8739 | 37 | 48 |
| | 11 | *Listeria monocytogenes* | ATCC 7644 ^{C} | 37 | 24 |
| | 12 | *L. harmless* | DSM 20649 | 37 | 24 |
| | 13 | *L. seeligeri* | DSM 20751 | 37 | 24 |
| | 14 | *L. marthii* | DSM 23813 | 37 | 24 |
| | 15 | *I walshimeri* | DSM 15452 | 37 | 24 |
| | 16 | *I ivanovii* | DSM 52491 | 37 | 24 |
| | 17 | *Staphylococcus aureus* | DI4A ^{D} | 37 | 24 |
| | 18 | *Bacillus cereus* | DSM 4282 | 37 | 24 |
| | 19 | *B. cereus* | DI4A RC3 | 37 | 24 |
| | 20 | *B. subtilis* | DSM 4181 | 30 | 24 |
| | 21 | *Salmonella enterica* | DSM 9378 | 37 | 24 |
| | 22 | *Escherichia coli* | DISTAM ^{E} | 37 | 24 |
| | 23 | *Lactobacillus plantarum* | ATCC RAA 793 | 30 | 24 |
| | 24 | *Saccharomyces cerevisiae* | ATCC 36024 | 30 | 48 |

Due to its specificity for the four *Campylobacter* species (*C*. *jejuni,* C*. coli,* C*. lari, C. upsaliensis),* the probe of SEQ ID NO 1 can be used for detecting these microorganisms in samples of interest using different methods known to a person skilled in the art. For this purpose, the probe or oligonucleotide can be bound, at 3' or 5' or at any position therein, to a molecule that allows its detection by such techniques. The invention therefore relates to the probe of SEQ ID NO 1, wherein said probe (preferably DNA) is labelled by binding at 3' to 5' or any position therein with a chemical reporter group, with an enzyme, with a fluorophore or with a thiol group (-SH) or an amine group (-NH₂).

According to the present invention, the chemical reporter group may for example be chosen from biotin, digoxigenin, these being recognized by avidin or streptavidin, and anti-Dig antibodies, which may be labelled with the enzyme, which may be alkaline phosphatase or peroxidase, glucosidase and ß-galactosidase; the fluorophore can be Cascade Blue (410), Pacific Blue (455), Oregon Green (488X), Bodipy FL-X (510), Fluorescene (520), 5.6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614) Bodipy TR-X (617), Light Cycler 640 (640), Bodipy 630/650-x (650), Cy5 (667) and Cy5.5 (694).

The label attached to the probe allows it to be used by means of the detection techniques considered most appropriate depending on the sample analyzed and the means available to the user.

The invention therefore relates to a method for detecting one or more of *C*. *jejuni, C. coli, C. lari* and *C*. *upsaliensis* in a sample comprising
a step of DNA extracting from said sample
a step of hybridizing the DNA extracted from said sample with the nucleotide probe of SEQ ID NO 1 according to any of the embodiments described herein
a step of detecting the hybridization of said probe to said DNA, in which the detection of a hybridization of said probe to said DNA indicates the presence of at least one of *C. jejuni, C. coli, C. lari* and *C. upsaliensis* in said sample.

Optionally, the DNA extraction step described above may be preceded by one or more of the following
a step of bacterial enrichment in said sample,
a step of cell concentration.

According to the present invention, enrichment can be achieved by standard techniques, for example by subjecting said sample to culture in Bolton broth or Preston broth.

According to the present invention, this concentration step can be carried out by centrifugation or filtration.

If desired, these steps can be repeated.

Furthermore, according to an embodiment of the present invention, said DNA is treated with RNAse before said hybridization step.

The hybridization and detection steps can be carried out by commonly used techniques such as PCR, microarray, FISH, Southern blot, dot blot, qPCR, RT PCR, bioplexing or by using biosensors.

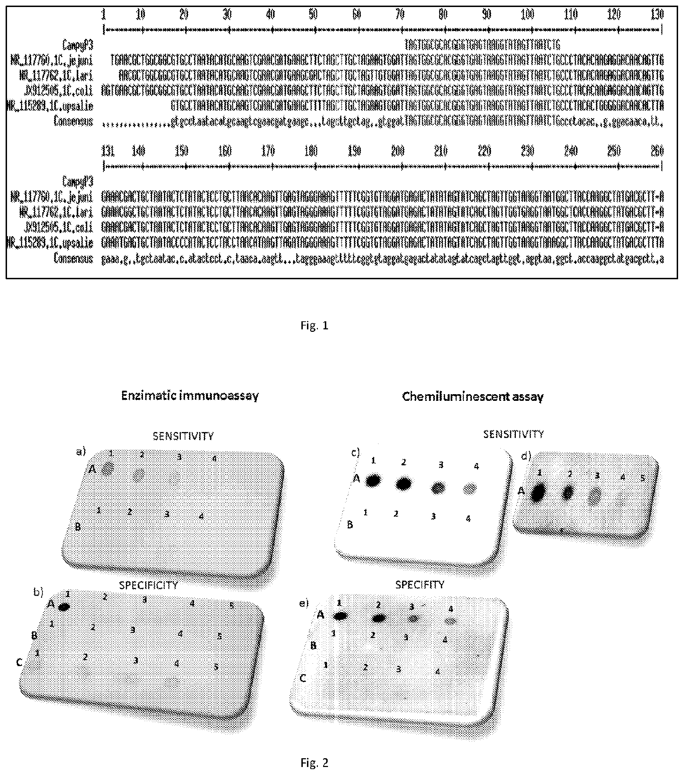

According to one embodiment of the present invention, the method may be carried out by performing hybridization and detection using PCR, qPCR or RT-PCR techniques. The PCR may be carried out using probes or oligonucleotides that amplify the coding region for the 16S rRNA or cDNA obtained from the mRNA comprising the probe sequence of the invention as shown in Figure 1. A person skilled in the art, starting from the known sequences of the gene in the microorganisms shown above and indicated in the claims will be able to design primers suitable for said amplification.

For example, the probe of SEQ ID NO 1 can be applied for the recognition of amplicons obtained by the PCR technique. The primers used may be universal primers for bacteria amplifying the 16S gene; the amplified region should include the sequence complementary to the above probe.

For example, known universal forward primers P1V1 having SEQ ID NO 2 and universal reverse primers 518R having SEQ ID NO 3 may be used. If the bacterium belongs to one of the four *Campylobacter* species set forth in the present description and claims, it can be identified by hybridization with the probe of the invention.

In a further embodiment, the probe of SEQ ID NO 1 can be used in the q-PCR technique as a fluorescent probe to quantify only the DNA containing the probe sequence, that is to say to quantify the presence of *Campylobacter* spp. in the sample. Again, universal primers that amplify the region of interest of the 16S RNA encoding gene in *Campylobacter* may be used, such as the pair of primers P1V1- 518R above, or they may be specifically designed to contain the probe, but they must always amplify the 16S gene. In this case, the probe of SEQ ID NO 1 should be labelled at 5' with a reporter and at 3' with a quencher.

Possible reporters are Cascade blue (410), Pacific blue (455), Oregon green (488X), Bodipy FL-X (510), Fluorescene (520), 5.6FAM (518), OregonGreen (524), TET (536), Bodipy R6G-X (547), JOE (548), HEX (556), Cy3 (570), Rhodamine Red-X (580), TAMARA (580), Cy3.5 (596), ROX (605), Texas Red-X (614) Bodipy TR-X (617), Light Cycler 640 (640), Bodipy 630/650-x (650), Cy5 (667) and Cy5.5 (694).

Possible quenchers can be those commonly used, such as Dabcyl, RHQ-1, QYS-7 and BHQ-2.O

According to a further embodiment, hybridization and detection can be carried out by means of Southern blotting or dot blot techniques.

In this case, genomic DNA extracted from the sample, optionally pre-treated with RNAse, is denatured and transferred to membranes according to standard protocols.

Hybridization with the probe (optionally labelled according to appropriate labelling techniques described here, and suitable for detection by blotting) is carried out under stringent hybridization conditions suitable for a probe that is 100% complementary.

A person skilled in the art will be able to easily deduce these conditions based on the chemical-physical characteristics of the probe given in this description and standard blotting procedures.

In a non-limiting embodiment, hybridization may be carried out at 65°C in an appropriate hybridization buffer, optionally by adding 2-5% formamide, and at different concentrations of SSC. Rinses may be carried out at the same temperature by gradually lowering the salt concentration, for example from 2XSSC, 1XSSC optionally down to 0.1XSSC.

Hybridization can be detected for example using a probe labelled with digoxigenin and using, for colorimetric detection, alkaline phosphatase or even chemiluminescence using horseradish peroxidase, or with a suitably labelled probe as indicated below.

Detection of the results of the hybridization with the probe of SEQ ID NO 1 to the DNA of the sample can therefore be carried out by enzymatic or fluorescence, immunoenzymatic, immunofluorescence, chemiluminescence, electrochemical (potentiometry, voltammetry, amperometry, impedance), piezoelectric, magnetic, photoacoustic, photothermal, thermal, radioactive and optical (colorimetry, LSPR, SPR, SERS, RAMAN, OLED, optical fibre) methods.

Accordingly, depending on the methodology chosen for hybridization and subsequent detection, the probe of SEQ ID NO 1 may be labelled, at 3' or 5' or internally, by binding a DNA base of said probe, with a chemical reporter group, with an enzyme, with a fluorophore or with a thiol group (-SH) or an amine group (NH₂) according to any of the embodiments described above.

The sample to be tested may be any sample in which the detection of *Campylobacter jejuni, Campylobacter coli, Campylobacter lari* or *Campylobacter upsaliensis* may be of interest, such as a food sample, an environmental sample and a biological sample.

An example of a food sample could be milk or dairy products of various kinds and fresh meat such as poultry, chicken, turkey, pork and beef.

An example of an environmental sample could be water or sewage, such as irrigation water, water or sewage taken from animal farms, or samples taken from breeding environments, premises dedicated to food processing or machinery for processing meat or other foods.

An example of a biological sample could be animal meat, human or animal faeces and blood (cattle, sheep, swans, pets) or human rectal swabs.

As mentioned above, the method of the invention can be carried out using hybridization and detection techniques such as PCR, blotting, microarray, FISH, Southern blot, dot blot, qPCR, RT-PCR, bioplexing or by using biosensors.

The invention thus also relates to a microarray comprising the probe of SEQ ID NO 1 and optionally further probes suitable for detecting pathogens.

The microarray can be realized according to any prior art teaching.

Biosensors are devices that convert a biological or physical event into a measurable signal. The biosensor consists of two components, a bioreceptor and a transducer. The first element has the function of recognizing the analyte, and thus the selectivity/specificity of the method depends on it. The second element has the function of transducing the biological signal, i.e. the interaction between analyte and bioreceptor, into an electrical signal. The analyte can be a nucleotide sequence, a cell, an antigen, a substrate or a tissue. The bioreceptor, which can be a nucleotide probe, an antibody, an enzyme or a cell, is chosen on the basis of what is being detected (Fig. 6).

The choice of transducer, which can be optical, piezoelectric or electrochemical, depends on sensitivity.

According to the present invention, the probe of SEQ ID NO 1 may be an unlabelled probe (capture probe) applicable to the following kinds of biosensors:
- optical such as SPR (Surface Plasmon Resonance), LSPR (Localized surface plasmon resonance), fibre optics, SERS (Surface Enhanced Raman Scattering), OLED (Organic Hight Emitting Diodes) and multiplex.
- piezoelectric such as quartz crystal microbalance (QCM)
- electrochemical based on amperometry, potentiometry, impedance and on organic electrochemical transistor.

Or, the probe may be a labelled probe (detection probe):
usable as a secondary probe in "sandwich" systems such as OLEDs.

It is therefore also an object of the present invention to provide a biosensor electrode on which oligonucleotide probes of SEQ ID NO 1 are immobilized according to any one of the embodiments described herein, and a biosensor comprising said electrode. Such a biosensor could be, for example, an optical biosensor, an electrochemical biosensor or a piezoelectric biosensor.

Non-limiting examples of optical biosensors are LSPR, SPR, RAMAN and SERS spectroscopy or OLED and fibre optic biosensors.

Functionalized nanoparticles (NPs) of various noble metals can be used for LSPR biosensors. For the fibre-optic technique, the probe of SEQ ID NO 1 can be used, labelled, for example, with biotin, conjugated with streptavidin bound to horseradish peroxidase, for a chemiluminescence system, or with Cy5 for a fluorescence system. Non-limiting examples of electrochemical biosensors include OECT (organic electrochemical transistors) in which an electrode or conductive polymer can be functionalized with the probe of SEQ ID NO 1.

The marking of the probe of SEQ ID NO 1 can be at terminal 3'or 5' or any position within it.

The type of labelling depends on the detection system as shown in Table 5.

**Table 5. Types of nucleotide probe labels.**

| **Probe label** | **Detection systems** |
|---|---|
| Digoxigenin: | Colorimetric Chemiluminescence |
| Ab anti-Dig labelled with ALP /HRP | |
| Fluorophore | Fluorescence |
| Biotin: | Colorimetric Chemiluminescence |
| Avidin/ streptavidin labelled with HRP/ALP/TMB | |

The invention also relates to a kit comprising aliquots of oligonucleotide probes of SE1 ID NO 1 and one or more reagents for their detection.

Depending on the embodiment, these probes can be labelled with a chemical reporter group, a fluorophore or a thiol group (-SH) or an amine group (-NH₂) as described above.

Lastly, the invention relates to a use of the kit, of the electrode, of the sensor or of the probe as defined in the present description and claims for detecting *C. jejuni, C. coli, C. lari* and *C*. *upsaliensis* in a sample.

In an exemplary embodiment, the probe, electrode, sensor and method of the invention, in any embodiment provided in the present description and claims, may be used in:
the food sector to increase the level of safety, through the control and management of critical points in the production, processing and distribution phases.
the environmental sector, insofar as *Campylobacter* can be detected in lakes and streams used as irrigation sources for fields.
the veterinary sector insofar as it may cause in animals, including cattle, sheep, goats and pigs, infections of the genital system (abortions, sterility), mammary system (mastitis) and digestive system (enteritis, hepatitis). The detection of *Campylobacter* is especially important in poultry and pig farms because:
   - a decision can be made to undertake antibiotic treatment where necessary, preventing the development of a form of resistance by the pathogen.
   - these pathogens can act as a source of contamination for food and may pose a risk to operators
the clinical field by speeding up diagnosis and deciding on antibiotic treatment for those most at risk and for patients with extra-intestinal infections to prevent recurrence.

The following examples are intended to indicate possible embodiments of the invention and provide experimental data obtained in the characterization of the probe of SEQ ID NO 1.

### EXAMPLES

### 1. PROTOCOLS AND RESULTS OF THE VARIOUS DETECTION SYSTEMS TO WHICH THE CAMPYP3 PROBE WAS APPLIED (SEQ ID NO 1)

### 1. 1 Preparation of DNA samples of the micro-organisms tested for sensitivity and specificity testing of the CampyP3 probe

The microorganisms used to test the specificity of the probe are listed in Table 4.

The positive controls, i.e. *Campylobacter* species, corresponding to numbers 1,2,3,4 were revitalized in BHI (Brain Heart Infusion) broth (Oxoid, Milan, Italy).

Subsequently, *Campylobacter* spp. was isolated on Columbia blood agar medium to which 5% defibrinated sheep blood (Oxoid, Milan, Italy) was added.

Once the morphological correspondence of the colony had been confirmed, it was isolated again on BHI agar medium to perform the various identification tests such as Gram stain, cell morphological characteristics, oxidase and catalase tests.

All incubation steps of *Campylobacter* spp. took place at 37°C for 48 hours under specific microaerophilic conditions (6% O₂, 7% CO₂, 7% H₂ and 80% N₂) which are generated with Sachet Oxoid^{™} CampyGen^{™} 2.5 L (Oxoid, Milan, Italy).

The negative controls, i.e. *Campylobacter fetus, C. cryaerophila, Helicobacter pylori, H. suis, Arcobacter butzleri* corresponding to nos. 5,6,7,8,9,10, were grown under specific micro-aerophilic conditions (6% O2, 7% CO₂, 7% H₂ and 80% N₂) generated by the Oxoid^{™} CampyGen^{™} 2.5 L Sachet. Incubation took place at 37°C for 48 hours (Oxoid, Milan, Italy).

*Lactobacillus plantarum,* corresponding to no. 23, was grown under standard microaerophilic conditions. The incubation temperature was 30°C for 24 hours.

By contrast, the remaining negative controls, i.e. *Listeria monocytogenes, L. innocua, L. seeligeri, L. marthii, L. welshimeri, L. ivanovii, Staphylococcus aureus, Bacillus cereus, B. cereus* RC3, *B. subtilis, Salmonella enterica, Escherichia coli* and *Saccharomyces cerevisiae,* corresponding to nos. 11 to 22 and 24 were grown under aerobic conditions. All controls were incubated at 37°C for 24 hours, except *B. subtilis, Lactobacillus plantarum* and *Saccharomyces cerevisiae,* corresponding to nos. 20, 23, and 24, which had an optimal growth temperature of 30°C for 24 hours or 48 hours (see Table 4 above).

All negative controls were revitalized in BHI broth and then isolated on BHI agar medium to perform the identification tests listed above, except for *S*. *cerevisiae,* which required Malt extract broth (Oxoid, Milan, Italy) for revitalization and Malt extract agar medium for isolation. For some bacterial species, an additional identification test was performed using appropriate selective media. The different *Listeria spp.* species were isolated on PALCAM Agar Base, S. *aureus* on Baird parker agar, *S. enterica* on X.L.D, agar and *Bacillus* spp. on Brilliance^{™} Bacillus Cereus Agar Base (Oxoid, Milan, Italy). Once the identification of the micro-organism had been confirmed, DNA extraction was performed.

### 1.1.1 Extraction of genomic DNA

### Reagent preparation

- Breaking Buffer: 2% Triton, 1% SDS, 100 mM NaCl, 10 mM tris base, 1 mM EDTA
- Lysozyme: 0.1g/mL lysozyme in 25% sucrose solution
- TE buffer: 10 mM Tris, 1 mM EDTA, pH 8.
- RNAse 0.01g/mL

Pure colonies of each bacterium were diluted in 2 mL of BHI broth and incubated at temperatures, times and conditions of aerobiosis or microaerobiosis specific to each bacterium.

For the yeast, a pure colony was diluted in 2 mL of Malt extract broth and incubated at 30°C for 48 hours.

The following steps were then performed:
1.Centrifuging the broth containing the cultures at 13,000 rpm for 10 minutes.
2.Discarding the supernatant
3.Resuspending the pellet in 300 µL of Breaking Buffer
4.Adding 30 µL of lysozyme (for yeasts and Gram-negative bacteria, this step is not necessary).
5.Transferring into test tubes containing glass beads with a diameter of 0.5 mm (previously prepared and sterilized).
6.Adding 300 µL of phenol-chloroform-isoamyl alcohol in a 24:25:1 ratio.
7.Stirring under vortex 3 times for 60 seconds each, with a 1-minute break between treatments
8.Adding 300 µL of TE and stirring gently to invert.
9.Centrifuging at 13,000 rpm for 10 minutes
10.Removing the supernatant, containing the DNA, whilst avoiding removal of the interphase protein film
11.Adding 1 mL of absolute ethanol at -20°C and stirring to invert
12.Centrifuging at 13,000 rpm for 10 minutes
13.Discarding the supernatant
14.Drying the DNA pellet under vacuum for 2 hours, or in a concentrator for 45 min, or in a thermostat at 37°C overnight
15.Resuspending the dehydrated DNA in 50 µL of sterile double-distilled H₂O
16.Adding 1 µL of RNase and incubating at 37°C for 1 hour.

### 1.1.2 Reading the concentration of the genomic DNA

The concentration of extracted DNA and its quality were read and analyzed on the NanodropTM 2000C spectrophotometer. All DNA samples were standardized to 100 ng/µL and stored at -20°C until use.

### 1.2 Application of the probe to varioust detection systems based on blotting methods.

### 1.2.1 Immunoenzymatic detection system

### Protocol for sensitivity testing

### Preparation of the CampyP3-Dig probe

- The CampyP3 probe was ordered from Az. Eurofins genomics modified by the addition of digoxigenin (Dig) at the 5' end and named CampyP3-Dig.
- The probe was rehydrated with sterile Milliq water with a volume specified by the company to obtain a concentration of 100 pmol/µL
- The probe was standardized at 100 ng/µL in sterile Milliq water and stored at -20°C until use. The concentration was checked on a spectrophotometer.

### Preparation of the CCP3 complementary probe (used as target DNA) for sensitivity testing

- The probe complementary to CampyP3, the CCP3 probe, was ordered from Eurofins genomics.
- The CCP3 probe was rehydrated with sterile Milliq water with a volume specified by the company to obtain a concentration of 100 pmol/µL
- The CCP3 probe was standardized, in sterile Milliq water, at different scalar concentrations: 100 ng/µL, 50 ng/µL, 10 ng/µL, 1 ng/µL, 0.1 ng/µL, 0.01 ng/µL, 0.001 ng/µL and 0.0001 ng/µL. Concentration, where possible, was checked on the spectrophotometer

### Preparation of reagents

- Maleic acid buffer 10X: prepare at 1X working concentration, dilute with sterile water (Roche, Monza, Italy)
- Blocking solution 1X: dilute blocking stock solution 10X (Roche, Monza, Italy) 1:10 in Maleic acid buffer. This solution should be prepared at the time of use.
- Ab- antiDIG: Centrifuge the tube containing Anti-Digoxigenin-AP (Roche, Monza, Italy) for 5 min at 10,000 rpm. Remove the aliquot from the supernatant and dilute 1:5,000 in 1X blocking solution. This solution should be prepared at the time of use.
- COLOR SUBSTRATE SOLUTION: Add 100 µL of NBT/BCIP (Roche) to 5 mL of DETECTION BUFFER ((Roche, Monza, Italy)) already diluted with 1X sterile water. This solution should be prepared shortly before use and should be protected from light.
- SSC 2X, 0.1% SDS (Sigma-Aldrich, Milan, Italy): 10 mL of SSC 20X (Roche, Monza, Italy) in 90 mL of double-distilled water and 0.1 g SDS, autoclave.
- SSC 0.5X, 0.1% SDS: 2.5 mL of 20X SSC in 97.5 mL of double-distilled water and 0.1 g SDS, autoclave.

### 1. HYBRIDIZATION

1.1Denature the samples (the different concentrations of CCP3) at 95°C for 10 min and place immediately on ice.

1.2. Deposit 1 µL of each sample onto the positively charged nylon membrane.

1.3. Fix DNA by UV light (254nm) for 10 min

1.4. Preheat 14 mL of Dig Easy Hyb buffer (Roche) in a water bath to a hybridization temperature of 65°C.

1.5. Pour 7 mL of pre-warmed Dig Easy Hyb buffer onto the membrane and incubate at 65°C with stirring for 30 min.

1.6. Denature 7 µL of CampyP3-Dig probe, standardized to 100 ng/µL, at 95° for 10 min and place immediately on ice.

1.7. Add 7 µL of CampyP3-Dig probe to the remaining 7 mL of Dig Easy Hyb buffer.

1.8. Discard 7 mL of Dig Easy Hyb buffer and add 7 mL of Dig Easy Hyb buffer with the denatured probe.

1.9. Leave the membrane to hybridize overnight with stirring at 65°C.

### 2. WASHING

### Discard the previous reagent with each wash step.

2.1 Wash the membrane twice for 10 min with 7 mL of 1X SSC with 0.1% SDS at room temperature and with stirring.

2.2 Wash the membrane twice for 15 min with 7 mL of 0.1X SSC with 0.1% SDS at room temperature and with stirring.

2.3 Wash the membrane with 7 mL of 1X Washing buffer for 5' at room temperature and with stirring.

### 3. IMMUNOENZYMATIC DETECTION

### Discard the previous reagent in each step.

3.1 Incubate the membrane with 10 mL of 1X blocking solution at room temperature and with stirring.

3.2 Incubate the membrane with 10 mL of 1X blocking solution with 1:5,000 diluted Ab anti-Dig at room temperature with stirring.

3.3 Wash twice for 15 min with 7 mL of 1X Washing buffer at room temperature and with stirring.

3.4 Equilibrate the membrane with 7 mL of 1X Detection Buffer for 5 min with stirring. 3.5 Incubate the membrane with 5 mL of colour substrate solution at room temperature, without stirring and protected from light.

3.6 Every 5 minutes check for the appearance of purple spots for 45 min.

### Protocol for the specificity test

### Preparation of the CampyP3-Dig probe

The CampyP3 probe was standardized at 100 ng/µL in sterile Milliq water and stored at -20°C until use. Its concentration was checked on a spectrophotometer.

### Preparation of the CCP3 probe

The CCP3 probe was standardized at 100 ng/µL, 50 ng/µL, 10 ng/µL, 1 ng/µL, 0.1 ng/µL, 0.01 ng/µL, 0.001 ng/µL, 0.0001 ng/µL in sterile Milliq water and stored at - 20°C until use. Concentrations, as far as possible, were checked by spectrophotometer.

### Preparation of bacterial DNA samples

Previously extracted *C. jejuni, C.coli, C. lari, C. upsaliensis, C. fetus, H. pylori, Arc butzleri and S. enterica* DNAs were standardized at 100ng/ ng/µL.

In addition, samples of *C. jejuni, C. coli, C. lari, C. upsaliensis and C. fetus* were used at the respective unstandardized concentrations of 256 ng/µL, 206 ng/µL, 612 ng/µL, 386 ng/µL and 218 ng/µL.

The protocol applied was the same as that used for the sensitivity test.

### 1.2.2 Chemiluminescence detection system

### Protocol for sensitivity testing

### Preparation of the CampyP3-Bio probe

- The CampyP3 probe was ordered from Az. Eurofins genomics modified with the addition of biotin at the 5' end and was named CampyP3-Bio.
- The probe was rehydrated with sterile Milliq water in a volume specified by the company.
- The probe was standardized at 100 ng/µL in sterile Milliq water and stored at -20°C until use. Its concentration was checked by spectrophotometer.

### Preparation of CCP3 complementary probe

- The probe complementary to CampyP3, the CCP3 probe, was ordered from Eurofins genomics.
- The CCP3 probe was rehydrated with sterile Milliq water in a volume specified by the company.

The CCP3 probe was standardized, in sterile Milliq water, at different scalar concentrations: 1 ng/µL, 0.5 ng/µL, 0.2 ng/µL, 0.1 ng/µL, 0.05 ng/µL, 0.02 ng/µL, 0.01 ng/µL and 0.005 ng/µL.

### Preparation of a DNA sample of C. jejuni

- DNA from the previously extracted *C. jejuni was* standardized at different concentrations: 100 ng/µL, 50 ng/µL, 10 ng/µL, 5 ng/µL and 1 ng/µL. The DNA concentrations were checked by spectrophotometer.

### Reagent preparation and experimental conditions

- hybridization buffer: 0.5M Na₂HPO4, 0.5M NaH₂PO₄, 10mM EDTA, 1%SDS at pH 7.5
- washing Buffer (Meraudex, France). SSC 2X + 0.1% SDS, SSC 0.5X + 0.1% SDS
- 1X wash solution (Thermofisher ^{Tm}, France): 40 mL 4X wash buffer + 120 mL sterile Milliq
- stirring 55 rpm

### Key

| |
|---|
| SSC: Sodium chloride sodium citrate |
| SDS: Sodium dodecyl sulphate |
| Blocking solution: with protein to saturate the non-specific site. |
| SSHPC: Stabilized horseradish streptavidin-peroxidase conjugate |
| Hyb °C: Hybridization temperature (probe-specific) |
| RT: ambient temperature |

### DAY 1

| 1 | HYBRIDIZATION | |
|---|---|---|
| 1.1 | Heat the hybridization buffer before use (prepare 2 tubes of 4 mL each) | 65°C |
| 1.2 | Denature the DNA and place immediately on ice | 95°C x 10 min |
| 1.3 | Deposit 1µL of denatured DNA onto positively charged nylon membrane | |
| 1.4 | Keep the membrane under UV (254 nm) to immobilize the DNA by formation of thymine dimers | 10 min |
| 1.5 | Prehybridization: immerse the membrane in the hybridization buffer | 65°C x 30 min, with stirring at 55 rpm |
| 1.6 | Denature the standardized CampyP3-Bio probe at 100 ng/µL and place immediately on ice. | 95°C x 5 min, |
| 1.7 | Hybridization: add 4µL of denatured CampyP3-Bio probe to 4mL of hybridization buffer | 65°C, overnight, with stirring at 55 rpm, close with parafilm |

### DAY 2

| Heat the following reagents SSC (65°C), blocking solution and washing buffer (37°C) in a bain-marie to dissolve | | |
|---|---|---|
| 2 | WASHING | |
| 2.1a | Wash the membrane in 4mL SSC 2X + 0.1% SDS in a new Petri dish | 65°C x 5 min, with stirring |
| 2.1b | Wash the membrane with 4mL SSC 2X + 0.1% SDS in a Petri dish | 65°C x5 min, with stirring |
| 2.2a | " " 4mL SSC 0.5X +0.1% SDS in a new Petri dish | 65°C x 15 min, with stirring |
| 2.2b | " " 4mL SSC 0.5X +0.1% SDS in a new Petri dish | 65°C x 15 min, with stirring |
| 2.3 | " " 3mLwashing buffer 1X in a new Petri dish | RTx 5 min, with stirring |

| 3 | INCUBATION | |
|---|---|---|
| 3.1 | Incubate membrane in 3mL blocking solution | RTx 15 min, with stirring |
| 3.2 | " " " * 3mL blocking solution + 5µL SSHPC (20 X) (EMD millipore corp., France). | RTx 15 min, with stirring |
| 3.3 | Transfer the membrane into a new Petri dish | |
| 3.3a | Wash the membrane with 3mL *wash solution 1X in a Petri dish | RT x 5 min |
| 3.3b | Wash the membrane with 3mL *wash solution 1X in a Petri dish | RT x 5 min |
| | Transfer the membrane into a new Petri dish | |

| 4 | DETECTION | |
|---|---|---|
| 4.1 | Pre-treat the membrane with 3mL Substrate Equilibration Buffer (Thermofisher^{™}, France) | RTx 5 min, with stirring |
| 4.2 | Prepare fresh Substrate working solution (1:1) 500 µL Luminol/ Enhancer Solution Buffer+ 500 µL Stable Peroxide Solution | |
| 4.3 | Remove excess buffer Transfer the membrane into a new Petri dish | 2-5 sec |
| 4.4 | Pour Substrate working solution onto the membrane (1mL) | RTx 1 min, |
| 4.5 | Remove the membrane from the solution, remove the excess, wrap the still wet membrane in see-through film | 2-5 sec |
| 4.6 | Expose the membrane to the CCD camera for X-ray film (ChemiDocTM MP Imaging System) | 2-5 min |
| 4.7 | Process data using Image LabTM Software (Biorad) | |

### Protocol for specificity testing

### Preparation of the CampyP3-Bio probe

The CampyP3-Bio probe was standardized at 100 ng/µL in sterile Milliq water and stored at -20°C until use. The probe concentration was checked on a spectrophotometer.

### Preparation of the CCP3 probe complementary

The CCP3 probe was standardized, in sterile Milliq water, at different concentrations: 1 ng/µL, 0.5 ng/µL, 0.2 ng/µL, 0.1 ng/µL, 0.05 ng/µL, 0.02 ng/µL, 0.01 ng/µL and 0.005 ng/µL.

### Preparation of nucleotide probes (PR and PE) acting as negative controls.

- Complementary probes similar to the CampyP3 probe sequence were designed to be used as negative controls and were named PR and PE.
- The PR and PE probes were ordered from a French company.
- The probes were rehydrated with sterile Milliq water in a volume specified by the company.
- The PR probe was standardized, in sterile Milliq water, at different concentrations: 1 ng/µL, 0.5 ng/µL, 0.2 ng/µL, 0.1 ng/µL, 0.05 ng/µL, 0.02 ng/µL, 0.01 ng/µL and 0.005 ng/µL and stored at -20°C until use.
- The PE probe was standardized, in sterile Milliq water, at different concentrations: 1 ng/µL, 0.5 ng/µL, 0.2 ng/µL, 0.1 ng/µL, 0.05 ng/µL, 0.02 ng/µL, 0.01 ng/µL and 0.005 ng/µL and stored at -20°C until use.

### Reagent preparation and experiment conditions

- hybridization buffer: 0.5M Na₂HPO4, 0.5M NaH₂PO₄, 10mM EDTA, 1%SDS at pH 7.5
- washing Buffer (Meraudex, France). SSC 2X + 0.1% SDS, SSC 0.5X + 0.1% SDS
- 1X wash solution (Thermofisher ^{Tm}, France): 40 mL 4X Wash Buffer + 120 mL Milliq
- stirring 55 rpm

The protocol applied is the same as that used for the sensitivity test.

### 1.2.3 New chemiluminescence-SiNPs detection system

### Protocol for sensitivity testing

### Preparation of the CampyP3-Bio probe

The CampyP3-Bio probe was standardized at 100 ng/µL in sterile Milliq water and stored at -20°C until use. The concentration of the probe was checked on a spectrophotometer.

### Preparation of CCP3complementary probe

The CCP3 probe was standardized, in sterile Milliq water, at different concentrations: 0.1 ng/µL, 0.05 ng/µL, 0.025 ng/µL, 0.0125 ng/µL, 0.006 ng/µL, 0.0031 ng/µL, 0.0015 ng/µL and 0.00078 ng/µL.

### Reagent preparation and experimental conditions

- hybridization buffer: 0.5M Na2HPO4, 0.5M NaH2PO4, 10mM EDTA, 1%SDS at pH 7.5
- washing Buffer (Meraudex, France): SSC 2X + 0.1% SDS, SSC 0.5X + 0.1% SDS
- 1X wash solution (Thermofisher ^{Tm}, France): 40 mL 4X Wash buffer + 120 mL Milliq
- stirring 55 rpm
- Prepare the SiNPs to a final concentration of 5x108 /mL in 1X PBS. Sonicate for 15 min before use.

### Key

| |
|---|
| SSC: Sodium chloride sodium citrate |
| SDS: Sodium dodecyl sulphate |
| Blocking solution: with protein to saturate the non-specific site. |
| SSHPC: Stabilized horseradish streptavidin-peroxidase conjugate |
| Hyb °C: Hybridization temperature (probe-specific) |
| RT: ambient temperature |

### DAY 1

| 1 | HYBRIDIZATION | |
|---|---|---|
| 1.1 | Heat the hybridization buffer before use (prepare 2 tubes of 4 mL each) | 65°C |
| 1.2 | Denature the DNA and place immediately on ice | 95°C x 10 min |
| 1.3 | Deposit 1µL of denatured DNA onto positively charged nylon membrane | |
| 1.4 | Keep the membrane under UV (254 nm) to immobilize the DNA and form thymine dimers | 10 min |
| 1.5 | Prehybridization: immerse the membrane in the hybridization buffer | 65°C x 30 min, with stirring at 55 rpm |
| 1.6 | Denature the standardized CampyP3-Bio probe at 100 ng/µL and place immediately on ice. | 95°C x 5 min, |
| 1.7 | Hybridization: add 4µL of denatured CampyP3-Bio probe to 4mL of hybridization buffer | 65°C, overnight, with stirring at 55 rpm, seal with film (parafilm) |

### DAY 2

| Heat the following reagents SSC (65°C), Blocking solution and washing buffer (37°C) in a bain-marie to dissolve | | |
|---|---|---|
| 2 | WASHING | |
| 2.1a | Wash the membrane with 4mL SSC 2X + 0.1% SDS in a new Petri dish | 65°C x 5 min, stirring |
| 2.1b | Wash the membrane with 4mL SSC 2X + 0.1% SDS in a Petri dish | 65°C x 5 min, stirring |
| 2.2a | " " 4mL SSC 0.5X +0.1% SDS in a new Petri dish | 65°C x 15 min, stirring |
| 2.2b | " " 4mL SSC 0.5X +0.1% SDS in a Petri dish | 65°C x 15 min, stirring |
| 2.3 | " " 3mLwashing buffer 1X in a new Petri dish | RT x 5 min, stirring |

| 3 | INCUBATION | |
|---|---|---|
| 3.1 | Incubate the membrane with 3mL Blocking solution | RT x 15 min, stirring |
| 3.2 | " " " * 3mL Blocking solution + 5µL SSHPC (20 X) (EMD millipore corp., France). | RT x 15 min, stirring |
| 3.3 | Transfer the membrane into a new Petri dish | |
| 3.3a | Wash the membrane with 3mL *wash solution 1X | RT x 5 min |
| 3.3b | Wash the membrane with 3mL *wash solution 1X | RTx 5 min |
| 3.4 | Transfer the membrane into a new Petri dish | |
| | Incubate the membrane with 7920µL PBS+ 80 µL SiNPs 5*108/mL inPBS (to obtain a final concentration of 5*106) | RT x 30 min, stirring |
| 3.5 | Transfer the membrane into a new Petri dish | |
| 3.5a | Wash the membrane with 4mL *wash solution 1X | RT x 5 min |
| 3.5b | Wash the membrane with 4mL *wash solution 1X | RT x 5 min |
| 3.6 | Transfer the membrane into a new Petri dish | |
| | Incubate the membrane with 3mL Blocking solution + 5µL SSHPC (20X) | RT x 15 min, stirring |
| 3.7 | Transfer the membrane into a new Petri dish | |
| 3.7a | Wash the membrane with 4mL *wash solution 1X | RT x 5 min |
| 3.7b | Wash the membrane with 4mL *wash solution 1X | RT x 5 min |
| | Transfer the membrane into a new Petri dish | |

| 4 | DETECTION | |
|---|---|---|
| 4.1 | Pre-treat the membrane with 3mL Substrate Equilibration Buffer (Thermofisher ^{Tm}, France) | RT x 5 min, stirring |
| 4.2 | Prepare fresh Substrate working solution (1:1) 500 µL Luminol/ Enhancer Solution Buffer+ 500 µL Stable Peroxide Solution | |
| 4.3 | Remove excess buffer Transfer the membrane into a new Petri dish | 2-5 sec |
| 4.4 | Pour Substrate working solution onto the membrane (1mL) | RT x1 min, NO stirring |
| 4.5 | Remove the membrane from the solution, remove the excess, wrap the still wet membrane in see-through film | 2-5 sec |
| 4.6 | Expose the membrane to the CCD camera for X-ray film (ChemiDocTM MP Imaging System) | 2-5 min |
| 4.7 | Process data using Image LabTM Software (Biorad) | |

### Protocol for specificity testing

### Preparation of the CampyP3-Bio probe

The CampyP3-Bio probe was standardized at 100 ng/µL in sterile Milliq water and stored at -20°C until use. The probe concentration was checked on a spectrophotometer.

### Preparation of the DNA sample of some of the micro-organisms in Table 4.

All previously extracted DNAs *(Campylobacter jejuni subsp. jejuni, C. coli, C. lari subsp. lari, C. upsaliensis, C. cryaerophila , Helicobacter pylori ¹, H. pylori ², Arcobacter butzleri , Listeria monocytogenes, L. innocua, L. seeligeri, L. marthii, L. welshimeri, L. ivanovii, Staphylococcus aureus, Bacillus cereus, B. cereus RC3, B. subtilis, Salmonella enterica, Escherichia coli, Lactobacteri, B. subtilis, L. innocua. ivanovii, Staphylococcus aureus, Bacillus cereus, B. cereus RC3, B. subtilis, Salmonella enterica, Escherichia coli, Lactobacillus plantarum* and *Saccharomyces cerevisiae),* were standardized at 100 ng/µL in sterile Milliq water and stored at -20°C until use.

### Reagent preparation and experimental conditions

hybridization buffer: 0.5M NaH₂PO₄, 10mM EDTA, 1%SDS at pH 7.5
- washing Buffer (Meraudex, France). SSC 2X + 0.1% SDS, SSC 0.5X + 0.1% SDS
- 1X wash solution (Thermofisher ^{Tm}, France): 40 mL 4X Wash buffer + 120 mL Milliq
- stirring 55 rpm
- Prepare the SiNPs to a final concentration of 5 x10⁸ /mL in 1X PBS. Sonicate for 15 min before use.

The protocol applied is the same as that used for the sensitivity test.

### 1.2.4 Results obtained with blotting systems

Figure 2 shows the data obtained with the CampyP3-Dig probe applied to the immunoenzymatic system.

Membrane a shows the sensitivity test data. In line A and B the CCP3 probe is deposited at scalar concentrations. At position A4 the last visible spot is present. This indicates that the detection limit is 1 ng/µL.

Membrane b shows the specificity test data.

The spot of the CCP3 control is present in line A at position A1. From position A2 to A5, the various negative controls were deposited at a concentration of 100 ng/µL, and, correctly, no spot was detected, as the CampyP3-Dig probe did not hybridize to the DNA.

In line B, the different *Campylobacter* species standardized to 100 ng/µL were deposited, and only the spot for *C*. *jejuni* was detected at position B1.

By contrast, in line C, the different *Campylobacter* species were deposited at higher concentrations. The test correctly presented positivity for *C. jejuni, C. lari, C. coli* and *C. upsaliensis,* but not for *C*. *fetus.*

The data shown demonstrate the specificity of the probe.

Membranes *c*, *d* and *e* show the data obtained with the CampyP3-Bio probe applied to the chemiluminescence-based system.

Membrane *c* shows the result of the sensitivity test. In line A and B the CCP3 probe was deposited at scalar concentrations. The last visible spot is present at position A4. This indicates that the detection limit with this system is 0.1 ng/µL.

In membrane *d*, the sensitivity test was also conducted with genomic DNA from *C*. *jejuni.* Different scalar concentrations of DNA were deposited in the A-line. The detection limit for genomic DNA is 5 ng/µL.

Membrane *e* shows the specificity test data. Equal scalar concentrations were deposited in lines A, B and C for the CCP3, PR and PE probes, respectively. The CCP3 probe confirmed the data obtained in membrane *c*. The PR and PE probes, acting as negative controls, did not give a positive result.

The reported data show that the chemiluminescence-based system increases sensitivity 10-fold for CCP3 probe detection and 20-fold for genomic DNA detection compared to the enzyme immunoassay.

The specificity of the CampyP3 probe is confirmed, even when applied to a more sensitive system. In fact, no positivity is obtained even when testing short DNA sequences with high similarity and which do not suffer from steric clutter compared to genomic DNA.

Membrane *a)* CCP3 in row A, A1: 100 ng / µL, A2: 50 ng / µL, A3: 10 ng / µL, A4: 1 ng / µL. CCP3 in row B, B1: 0.1 ng / µL B2: 0.01 ng / µL B3: 0.001 ng / µL B4: 0.0001 ng / µL. Membrane *b*) row A, A1: CCP3 100 ng / µL, A2: *E. coli* 100 ng / µL, A3: *H. pylori* 100 ng / µL, A4: *Arc. butzleri* 100 ng / µL A5: *S. enterica* 100 ng / µL. row B, B1: *C. jejuni* 100 ng / µL B2: *C. coli* 100 ng / µL B3: *C. lari100* ng / µL B4: *C. upsaliensis* ng / µL B5: *C. fetus* 100 ng / µL. row C, C1: *C. jejuni* 256 ng / µL C2: *C. coli* 206 ng / µL C3: *C. lari* 612 ng / µL C4: *C. upsaliensis* 386 ng / µL C5: *C. fetus* 218 ng / µL. Membrane *c)* row A, A1: CCP3 1 ng / µL A2: CCP3 0.5 ng / µL A3: CCP3 0.2 ng / µL A4: CCP3 0.1 ng / µL. row B, B1: CCP3 0.05 ng / µL, B2: CCP3 0.02 ng / µL, B3: CCP3 0.01 ng / µL, B4: CCP3 0.005 ng / µL Membrane *d*) row A, A1: *C. jejuni* 100 ng / µL A2 : *C. jejuni* 50 ng / µL A3: *C. jejuni* 10 ng / µL A4: *C. jejuni* 5 ng / µL A5: *C. jejuni* 1 ng / µL Membrane *e*) CCP3 in row A A1: 0.8 ng / µL A2: 0.4 ng / µL A3: 0.2 ng / µL A4 0.1 ng / µL. PR in row B: B1: 0.8 ng / µL B2: 0.4 ng / µL b3: 0.2 ng / µL b4 0.1 ng / µL. PE in row C C1: 0.8 ng / µL, C2: 0.4 ng / µL, C3: 0.2 ng / µL, C4 0.1 ng / µL.

Figure 3a shows the data of the sensitivity test with the chemiluminescence method in membrane *a* and the data of the sensitivity test with the system based on chemiluminescence-SiNPs in membrane *b.* The deposited concentrations of the CCP3 probe for both methods were the same.

In membrane a the detection limit is 0.1 ng/µL and is visible at position A1. The result confirms the detection limit already obtained in the previous experiment (Fig. 2c and Fig. 2e line A). In membrane *b* the detection limit is visible at position B1 and is 0.006 ng/µL.

In membrane *c,* the signal intensity obtained between the two methods is compared. The graph confirms the higher sensitivity of the system based on chemiluminescence-SiNPs, which increased 16-fold compared to the chemiluminescence method and 166-fold compared to the method based on the immunoenzymatic system.

Figure 3 shows the chemiluminescent results without and with NPs tested with CCP3 complementary probe. Membrane *a*) row A, A1: 0.1 ng / µL, A2: 0.05 ng / µL, A3: 0.025 ng / µL, A4: 0.0125 ng / µL; row B B1: 0.006 ng / µL, B2: 0.0031 ng / µL, B3: 0.0015 ng / µL, B4 0.00078 ng / µL. Membrane (*b)* row A, A1 0.1 ng / µL, A2: 0.05 ng / µL, A3: 0.025 ng / µL, A4: 0.0125 ng / µL; row B, B1: 0.006 ng / µL, B2: 0.0031 ng / µL, B3: 0.0015 ng / µL, B4 0.00078 ng / µL. (*c)* Comparison between the value obtained without NPs (low curve) and with NPs (high curve).

Figure 4 shows the results of the specificity test in which DNAs from different microorganisms were tested (Table 4). Signal intensity measurements were repeated three times. The average of the values are shown in the following graph. The graph shows the difference in signal intensity between the positive controls in dark grey and the negative samples in light grey. The data shown demonstrate the specificity of the probe.

### 1.3 Application of the probe to an electrochemical biosensor (Screen printed Au electrode, SPAuE).

**Protocol for constructing the calibration curve and for testing sensitivity and specificity.**

### Probe preparation

- The CampyP3 probe was ordered from Az. Eurofins genomics modified by the addition of the thiol group (SH) at the 5' end to allow its immobilization on a gold electrode. The probe was named CampyP3-SH
- The CampyP3-SH probe must be deprotected using the following protocol before use. Add 200uL of 10mM TECP (2.9 mg TCEP-HCI in 1mL of 1X TE buffer, pH 8) to the dehydrated probe. Stir for 1 hour at room temperature. Precipitate the probe by adding 150 uL of 3M NaAc (24.6g NaAc and 0.21g in 100 mL of Milliq), then add ethanol. Mix gently and incubate for 20 min at -20°C. Centrifuge for 5 min at 13,000rpm. Discard the supernatant and dry the pellet at room temperature. Rehydrate the probe with the volume predetermined by Az. Eurofins.
- The probe is standardized at 10 ng/µL in PBS 1X and stored at -20°C until used. Preparation of CCP3 complementary probe (used as target DNA) for sensitivity testing
- The probe complementary to CampyP3, the CCP3 probe, was ordered from Eurofins genomics.
- The CCP3 probe was rehydrated with sterile Milliq water with a volume specified by the company to obtain the final concentration of 100 pmol/µL.
- The CCP3 probe was standardized, in sterile Milliq water, at different scalar concentrations: 10,000 pg/µL, 1,000 pg/µL, 100 pg/µL, 10 pg/µL and 1 pg/µL.

### Preparation of microorganism DNAs for specificity and sensitivity testing

- DNA of *C. jejuni, C. fetus, E. coli, L. innocua,* previously extracted, was standardized, in sterile Milliq water, at different scalar concentrations: 10,000 pg/µL, 1,000 pg/µL, 100 pg/µL, 10 pg/µL and 1 pg/µL.

### Preparation of reagents

- H₂SO₄ 0.5 M (96% sulphuric acid) prepared in sterile double-distilled water (Carlo Erba, Milan, Italy).
- PBS_table, 1X buffer (0.0027 M KCI and 0.137 M NaCl, pH 7.4), solubilized in 200 mL of sterile double-distilled water (Sigma-Aldrich, Milan, Italy).
- 6-mercapto-1-hexanol (MCH) at 10 mM, (Sigma- Aldrich, Milan, Italy), solubilized in anhydrous absolute ethanol (Carlo Erba, Milan, Italy) then the 1 mM MCH solution was prepared in sterile PBS buffer.
- Potassium ferrocyanide (k₄[Fe(CN)6-3H₂O]) 10 mM in sterile 1X PBS buffer (AnalytiCals, Milan, Italy)

### Equipment

- electrochemical analysis was performed using PGSTAT101 autolab potentiostat galvanostat and NOVA 2.1 software (Methrom-Autolab B.V., Netherlands)
- screen-printed gold electrodes (SPAuEs) DropSens 220 AT (Au-Au-Ag/AgCl) (Metrohm- DropSens, Spain) were used. The electrochemical cell consisted of a gold working electrode (WE), a gold auxiliary electrode (CE) and a silver reference electrode (RE). The working area had a diameter of 4 mm.

### Conditioning program

| | |
|---|---|
| Initial potential | 0 V |
| Maximum potential | 1.3 V |
| Minimum potential | -0.002 V |
| End Potential | 0 V |
| Number of scans | 10 |
| Scanning rate | 0.1 V/s |
| Step | 0.00198 V |

### Differential pulse voltammetry (DPV) measurement program

| | |
|---|---|
| Initial potential | -0.2 V |
| End Potential | 0.4 V |
| Step | 0.005 V |
| Amplitude modulation | 0.15 V |
| Modulation time | 0.05s |
| Interval time | 0.5s |
| Scanning rate | 0.01 V/s |

| | STEP | | |
|---|---|---|---|
| **1** | **Electrode conditioning** | | |
| | Connect the SPAuE to the potentiostat/galvanostat | | |
| | Immerse SPAuE in 8 mL of 0.5M H₂SO₄. | | |
| | Apply the conditioning program | | |
| | Rinse the electrode with 500 uL of sterile H₂O | X 2 times | |
| | Dry under the laminar flow hood | | |
| **2** | **Immobilization of the CampyP3-SH probe** | | |
| | Denature 12 uL of probe at 10 ng/uL | 95°C x 10 min | |
| | Deposit 12uL of probe on the working electrode | Over-night at 25°C | |
| | Wash the surface with 500uL of sterile H₂O | X 2 times | |
| | Dry under the laminar flow hood | | |
| **3** | **Surface blocking** | | |
| | Deposit 12 uL of MCH 1mM on the working electrode | 1 hour at 25°C | |
| | Wash the surface with 500uL of H₂O | X 2 times | |
| | Dry under the laminar flow hood | | |
| **4** | **DPV measurement (differential pulse voltammetry)** | | |
| | Deposit 80uL of FeCH (k₄[Fe(CN)6-3H₂O] )10 mM | | |
| | Apply the DPV measurement program | | |
| | Wash the surface with 500uL of sterile H₂O | | |
| | Dry under the laminar flow hood | | |
| **5** | **DNA sample hybridization** | | Steps 5 and 6 are repeated for each DNA concentration to be tested to create the calibration curve. In this case for 10,000 pg/µL, 1,000 pg/µL, 100 pg/µL, 10 pg/µL and 1 pg/µL of the CCP3 probe and the different microorganisms. |
| | Denature the sample | 95°C x 10 min | |
| | Deposit 12 uL on the working electrode | 1 hour at 25°C | |
| | Wash the surface with 500uL of sterile H₂O | X 2 times | |
| | Dry under the laminar flow hood | | |
| **6** | **Measurement in DPV** | | |
| | Deposit 80uL of (k₄[Fe(CN)6-3H₂O])10 mM | | |
| | Apply the DPV measurement program | | |
| | Wash the surface with 500uL of sterile H₂O | X 2 times | |
| | Dry under the laminar flow hood | | |

### 1.3.1 Results

### Results for the system specificity and sensitivity test

Figure 5 shows the delta of the intensity of the anodic peak on the ordinate and the various DNA concentrations tested (pg/uL) on the abscissa expressed in logarithm. The calibration curves reported are for the following micro-organisms *C. jejuni, C. fetus, E. coli* and *L. monocytogenes.*

### Specificity assessment.

From Figure 5 it can be deduced that the intensity of the anodic peak decreases proportionally as the concentration of the target DNA, *C*. *jejuni,* hybridized to the probe increases (yellow regression line). However, as expected, there is no significant decrease in the intensity of the anodic peak proportional to the DNA concentrations for negative controls *(C. fetus, E. coli* and *L. innocua*). This is due to the fact that the DNA did not hybridize to the CampyP3-SH probe.

A parameter confirming the specificity of the system is the value of the gradient of each line: -21.591 for *C*. *jejuni,* -2.5257 for *C*. *fetus,* -4.181 for *L. innocua* and 6.299 for *E*. *coli.* The steepest gradient is, rightly, obtained with the genomic DNA of *C. jejuni.*

Another value to be taken into account is the intercept, which for *C. jejuni* is -14.316, for *C*. *fetus* is +3.7687, for *L. innocua* is +1.9773 and for *E. coli* is +9.563. The intercept value represents the decrease in current intensity at the lowest concentration tested. Again, the greatest decrease, at the same concentrations, is that for *C. jejuni.*

### Sensitivity assessment.

From the graph in Figure 5 it can be seen that the detection limit is 10 pg/µL, which is equal to 1 Log because the signal measured for the lowest concentration tested (1 pg/uL which is equal to 0 Log) is similar for the different micro-organisms, regardless of whether they are positive or negative controls.

### 2. ANALYSIS OF CHICKEN SAMPLES

### 2.1 Sample preparation

7 samples of fresh chicken meat were analyzed and named: 2SCB, 3SCB, 4SCB, 5SCB, 6SCB, 7SCB and 10SCB.

### Protocol

- Prepare a 1:10 suspension of the sample in the enrichment broth, i.e. Bolton Broth.
- Incubate at 37°C for 4-6 hours under specified microaerophilic conditions and then incubate at 41.5°C for 44 ± 4 hours under specified microaerophilic conditions.
- Remove 2 mL of DNA extraction broth,

### 2.2 DNA extraction from enrichment broth

### Reagent preparation

- Lysozyme: 0.1g/mL lysozyme in 25% sucrose solution
- Breaking Buffer: 2% Triton, 1% SDS, 100 mM NaCl, 10 mM tris base, 1 mM EDTA
- TE buffer: 10 mM Tris, 1 mM EDTA, pH 8.
- RNAse 0.01g/mL

1. Prepare a 1:10 suspension of the chicken sample in the enrichment broth, i.e. Bolton Broth.
2. Incubate at 37°C for 4-6 hours under specified microaerophilic conditions and then incubate at 41.5°C for 44 ± 4 hours under specified microaerophilic conditions.
3. Remove 2 mL of broth and centrifuge at 13,000 rpm for 10 minutes.
4.Discard the supernatant
5.Resuspend the pellet in 300 µL of Breaking Buffer
6.Add 30 µL of lysozyme
7.Transfer into test tubes containing glass beads with a diameter of 0.5 mm.
8.Add 300 µL of phenol-chloroform-isoamyl alcohol in a 24:25:1 ratio under a hood and wearing gloves
9.Stir 3 times under vortex for 60 seconds, with 2 minutes pause between treatments
10.Add 300 µL of TE and stir gently to invert.
11.Centrifuge at 13,000 rpm for 10 minutes
12.Remove the supernatant, containing the DNA, avoiding entrainment of the interphase protein film
13.Add 1 mL of absolute ethanol at -20°C and stir to invert
14.Centrifuge at 13,000 rpm for 10 minutes
15.Discard the supernatant
16.Dry the pellets under vacuum for 2 hours, or in a concentrator for 45 minutes, or in a thermostat at 37°C overnight.
17.Resuspend in 50 µL of sterile double-distilled H₂O
18.Add 1 µL of RNase and incubate at 37°C for 1 hour
20. Store the DNA at -20°C before use.

### 2.2 Analysis using the chemiluminescence-SiNPs system

### Preparation of the CampyP3 probe

The CampyP3-Bio probe was standardized at 100 ng/µL in sterile Milliq water and stored at -20°C until use.

### Preparation of the CCP3 complementary probe

- The CCP3 probe was standardized in sterile Milliq water at a concentration of 0.1 ng/µL,

### Preparation of chicken DNA sample.

5 chicken samples were analyzed and were named 3SCB, 4SCB, 5SCB, 6SCB and 10SCB.

### Reagent preparation and experimental conditions

- hybridization buffer: 0.5M Na₂HPO4, 0.5M NaH₂PO₄, 10mM EDTA, 1%SDS at pH 7.5
- washing Buffer (Meraudex, France). SSC 2X + 0.1% SDS, SSC 0.5X + 0.1% SDS
- 1X wash solution (Thermofisher ^{Tm}, France): 40 mL 4X Wash buffer + 120 mL Milliq
- stirring 55 rpm
- prepare SiNPs to a final concentration of 5 x10⁸ /mL in 1X PBS. Sonicate for 15 min before use.

### Key

| |
|---|
| SSC: Sodium chloride sodium citrate |
| SDS: Sodium dodecyl phosphate |
| Blocking solution: with proteins to saturate non-specific sites. |
| SSHPC: Stabilized Strepavidin-Horseradish Peroxidase Conjugate |
| Hyb °C: Hybridization temperatures (probe-specific) |
| RT: ambient temperature |

### DAY 1

| | | |
|---|---|---|
| 1 | HYBRIDATION | |
| 1.1 | Heat the hybridization buffer before use (prepare 2 tubes of 4 mL each) | 65°C |
| 1.2 | Denature the DNA and place immediately on ice | 95°C x 10 min |
| 1.3 | Spot 1µL of denatured DNA on positively charged nylon membrane | |
| 1.4 | Keep the membrane under UV (254 nm) to immobilize the DNA and form thymine dimers | 10 min |
| 1.5 | Pre-hybridization: immerse the membrane in the hybridization buffer | 65°C x 30 min, stirring |
| 1.6 | Denature the standardized CampyP3-Bio probe at 100 ng/µL and place immediately on ice. | 95°C x 5 min, |
| 1.7 | Hybridization: add 4µL of denatured CampyP3-Bio probe to 4mL of hybridization buffer | 65°C, overnight, stirring at 55 rpm, seal with film (parafilm) |

### DAY 2

| Heat the following reagents SSC (65°C), Blocking solution and washing buffer (37°C) in a bain-marie to dissolve | | |
|---|---|---|
| 2 | WASHING | |
| 2.1a | Wash the membrane with 4mL SSC 2X + 0.1% SDS in a new Petri dish | 65°C x 5 min, with stirring |
| 2.1b | Wash the membrane with 4mL SSC 2X + 0.1% SDS in a Petri dish | 65°C x 5 min, with stirring |
| 2.2a | " " 4mL SSC 0.5X +0.1% SDS in a new Petri dish | 65°C x 15 min, with stirring |
| 2.2b | " " 4mL SSC 0.5X +0.1% SDS in a Petri dish | 65°C x 15 min, with stirring |
| 2.3 | " " 3mL 1X washing buffer in a new Petri dish | RT x 5 min, with stirring |
| 3 | INCUBATION | |
| 3.1 | Incubate the membrane with 3mL Blocking solution | RT x15 min, with stirring |
| 3.2 | " " " * 3mL Blocking solution + 5µL SSHPC (20 X) (EMD millipore corp., France). | RT x 15 min, with stirring |
| 3.3 | Transfer the membrane into a new Petri dish | |
| 3.3a | Wash the membrane with 3mL *wash solution 1X | RT x 5 min |
| 3.3b | Wash the membrane with 3mL *wash solution 1X | RT x 5 min |
| 3.4 | Transfer the membrane into a new Petri dish | |
| | Incubate the membrane with 7920µL PBS+ 80 µL SiNPs 5*10⁸/mL inPBS (to obtain a final concentration of 5*106) | RT x 30 min, with stirring |
| 3.5 | Transfer the membrane into a new Petri dish | |
| 3.5a | Wash the membrane with 4mL *wash solution 1X in a new Petri dish | RT x 5 min |
| 3.5b | Wash the membrane with 4mL *wash solution 1X | RT x 5 min |
| 3.6 | Transfer the membrane into a new Petri dish | |
| | Incubate the membrane with 3mL blocking solution + 5µL SSHPC (20X) | RT x, 15 min, with stirring |
| 3.7 | Transfer the membrane into a new Petri dish | |
| 3.7a | Wash the membrane with 4mL *wash solution 1X | RT x 5 min |
| 3.7b | Wash the membrane with 4mL *wash solution 1X | RT x 5 min |
| | Transfer the membrane into a new Petri dish | |
| 4 | DETECTION | |
| 4.1 | Pre-treat the membrane with 3mL Substrate Equilibration Buffer (Thermofisher ^{Tm}, France) | RT x 5 min, with stirring |
| 4.2 | *Prepare fresh Substrate working solution (1:1) 500 µL Luminol/ Enhancer Solution Buffer+ 500 µL Stable Peroxide Solution | |
| 4.3 | Remove excess buffer | 2-5 sec |
| | Transfer the membrane into a new Petri dish | |
| 4.4 | Pour the Substrate working solution on the membrane (1mL) | RT x 1 min, NO stirring |
| 4.5 | Remove the membrane from the solution, remove the excess, wrap the still wet membrane in see-through film | 2-5 sec |
| 4.6 | Expose the membrane to the CCD camera for X-ray film (ChemiDocTM MP Imaging System) | 2-5 min |
| 4.7 | Process data using Image Lab^{™} Software (Biorad) | |

### 2.2.1 Results

The results obtained are shown in Figure 7.

In position A1 the positive control CCP3 can be seen. Positive results were obtained for the samples positioned at A2 and A3 and are samples 3SCB and 10SCB respectively. The samples positioned at B1, B2 and B3 correspond to 4SCB 5 SCB and 6SCB respectively and were negative.

### 2.3 Analysis with the electrochemical biosensor (Screen Printed Au Electrode, SPAuE)

### Preparation of the CampvP3-SH probe

The CampyP3-SH probe was standardized at 100 ng/µL in sterile Milliq water and stored at -20°C until use.

### Preparation of DNA from microorganisms

*E. coli* DNA was standardized, in sterile Milliq water, to: 10 ng/µL. Used as a negative control at the highest concentration.

### Preparation of chicken DNA sample

3 chicken samples were analyzed and named 2SCB, 3SCB and 7SCB.

| | STEP | |
|---|---|---|
| **1** | **Electrode conditioning** | |
| | Connect the SPAuE to the potentiostat/galvanostat | |
| | Immerse the SPAuE in 8 mL of 0.5M H₂SO₄. | |
| | Apply the conditioning program | |
| | Rinse the electrode with 500 uL of sterile H₂O | X 2 times |
| | Dry under the laminar flow hood | |

| **2** | **Immobilization of the CampyP3-SH probe** | |
|---|---|---|
| | Denature 12 uL of probe at 10 nq/uL | 95°C x 10 min |
| | Deposit 12uL of probe on the working electrode | Over-night at 25°C |
| | Wash the surface with 500uL of sterile H₂O | X 2 times |
| | Dry under the laminar flow hood | |

| **3** | **Surface blocking** | |
|---|---|---|
| | Deposit 12 uL of MCH 1mM on the working electrode | 1 hour at 25°C |
| | Wash the surface with 500uL of sterile H₂O | X 2 times |
| | Dry under the laminar flow hood | |

| **4** | **DPV measurement (differential pulse voltammetry)** | |
|---|---|---|
| | Deposit 80u of FeCH (k₄[Fe(CN)6-3H₂O]) | |
| | 10 mM | |
| | Apply the DPV measurement program | |
| | Wash the surface with 500uL of sterile H₂O | |
| | Dry under the laminar flow hood | |

| **5** | **DNA sample hybridization** | |
|---|---|---|
| | Denature the sample | 95°C x 10 min |
| | Deposit 12 uL on the working electrode | 1 hour at 25°C |
| | Wash the surface with 500uL of sterile H₂O | X 2 times |
| | Dry under the laminar flow hood | |

| **6** | **Measurement in DPV** | |
|---|---|---|
| | Deposit 80uL of (k₄[Fe(CN)6-3H₂O])10 mM | |
| | Apply the DPV measurement program | |
| | Wash the surface with 500uL of sterile H₂O | X 2 times |
| | Dry under the laminar flow hood | |

### 2.3.1 Results

The results are shown in Figure 8. The ordinate shows the delta of the current intensity of the anodic peak and the abscissa the DNA concentration expressed in logarithm. The *E. coli,* 2SCB, 3SCB and 7SCB samples were measured and the value was fitted to the previously obtained calibration curve (circles) with the various *C. jejuni* concentrations.

From the graph it can be seen that the samples that are positive are 2SCB and 3SCB. *E. coli* is negative as the measured value does not lie on the calibration line.

The 7SCB sample is not to be considered positive taking into account the detection limit of the system, which is 10 pg/µL despite the value falling on the calibration line.

**3. MICROBAL ANALYSIS OF CHICKEN SAMPLES: ISO 10272-1:2006 for validation of results obtained by chemiluminescence-SiNPs and electrochemical biosensor**

7 chicken samples were analyzed and named: 2SCB 3SCB 4SCB 5SCB 6SCB 7SCB 10SCB.

### Protocol

1. Prepare a 1:10 suspension of the sample in the enrichment broth, i.e. Bolton Broth.
2. Incubate at 37°C for 4-6 hours under specified microaerophilic conditions and then incubate at 41.5°C for 44 ± 4 hours under specified microaerophilic conditions.
3. Remove 10 µL and isolate on two selective media mCCDA and Skirrow with appropriate supplements and incubate at 41.5°C for 44 ± 4 hours in a microaerophilic atmosphere.
4. Observe for the presence of typical colonies; on mCCDA *Campylobacter* colonies are often flat and moist with a tendency to swarm and a metallic reflection.
5. Select from each plate at least 5 typical colonies for confirmation tests and for isolation on Columbia Blood Agar. Incubate at 41.5°C for 44 ± 4 hours in a microaerophilic atmosphere.
6. Proceed with the confirmation tests:
   - Motility testing
   - Oxidase test
   - Isolate one colony on Columbia blood agar and incubate at 25.1°C in microaerophilic conditions.
   - Isolate one colony on Columbia blood agar and incubate at 41.5°C under aerobacterial conditions.

### 3.1 Results

Table 5 shows the data from the chicken samples tested.

The results show that the samples positive for the presence of *Campylobacter* are 2SCB,3SCB,10SCB. On the other hand, samples 4SCB, 5SCB, 6SCB and 7SCB were negative insofar as the isolated microorganisms grew both aerobically and at 25°C, which are intolerable growth conditions for *Campylobacter* spp. characteristics. Therefore, the ISO 10272-1:2006 analysis applied to the 7 chicken samples confirmed the data obtained with both the DOT-BLOT based on chemiluminescence-SiNPs and the electrochemical biosensor.

**Table 6. Presence (+) or absence (-) of Campylobacter spp. assessed by ISO10272-1:2006.**

| **Samples** | **mCCDA** | **SKR** | **CAB** | **Confirmation medium CAB presence/absence** | | **Oxidase** | **Motility** |
|---|---|---|---|---|---|---|---|
| | | | | **41.5° aerobic** | **25°C microaerobic** | | |
| **2 SCB** | **+** | **-** | **+** | **-** | **-** | **+** | **+** |
| **3 SCB** | **+** | **-** | **+** | **-** | **-** | **+** | **+** |
| **4 SCB** | **+** | **-** | **+** | **+** | **+** | **+** | **-** |
| **5 SCB** | **+** | **-** | **+** | **+** | **+** | **+** | **-** |
| **6 SCB** | **+** | **-** | **+** | **+** | **+** | **+** | **-** |
| **7 SCB** | **+** | **-** | **+** | **+** | **+** | **+** | **-** |
| **10 SCB** | **+** | **-** | **+** | **-** | **-** | **+** | **+** |

### 4. CONCLUSION

Taking into account the tests performed, it can be stated that:
- The CampyP3 probe is specific for: *C. jejuni, C. coli, C. lari and C. upsaliensis.*

Note that no false positives were obtained with *E. coli,* which is normally present in chicken; or with pathogens such as *H. pylori, Arc. butzleri, C. fetus* and *C. cryaerophila,* which have a genome very similar to the target *Campylobacter* species.

The probe retains its specificity even when tested under the worst conditions. That is, with short complementary and similar DNA sequences.
- The CampyP3 probe is able to specifically recognize both pure target DNA and DNA extracted from complex matrices such as food samples. Therefore, it is not affected by possible interference caused by impurities in the food DNA sample.
- The data confirm that the probe can be applied to different systems, while maintaining its specificity.
- Probe sensitivity varies depending on the analysis system to which it is applied.

| | | |
|---|---|---|
| 1 | DOT BLOT | |
| | enzyme immunoassay | 1 ng/uL |
| | chemiluminescent | 0.1 ng/uL |
| | chemiluminescent-SiNPs | 0.006 nq/uL |
| 2 | ELECTROCHEMICAL BIOSENSOR | 0.01 ng/uL |

## Claims

1. A nucleotide probe of SEQ ID NO 1.

2. The nucleotide probe according to claim 1, wherein said probe is labelled with a chemical reporter group, with an enzyme, with a fluorophore or with a chemical group such as a thiol (-SH) or with an amino group (-NH₂).

3. The nucleotide probe according to claim 2, wherein said chemical reporter group is biotin or digoxigenin, said enzyme is alkaline phosphatase, peroxidase glucosidase or ß-galactosidase, and said fluorophore is Cascade Blue (410), Pacific Blue (455), Oregon Green (488X), Bodipy FL-X (510), Fluorescene (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614) Bodipy TR-X (617), Light Cycler 640 (640), Bodipy 630/650-x (650), Cy5 (667) or Cy5.5 (694).

4. A method for detecting one or more of *C. jejuni, C. coli, C. lari* and *C. upsaliensis* in a sample comprising
a step of DNA extracting from said sample
a step of hybridizing the DNA extracted from said sample with the nucleotide probe as defined in any one of claims 1 to 3 and
a step of detecting the hybridization of said probe to said DNA, in which
the detection of a hybridization of said probe to said DNA indicates the presence of at least one of *C. jejuni, C. coli, C. lari* and *C. upsaliensis* in said sample.

5. The method according to claim 4 wherein said DNA extraction step is preceded by one or more of the following
a step of bacterial enrichment in said sample, and/or
a step of concentration of bacterial cells by centrifugation and/or filtration,

6. The method according to claim 4 or 5, wherein said DNA is treated with RNAse before said hybridization step, and/or
wherein said hybridization and detection steps are carried out by PCR, blotting, microarray, FISH, Southern blot, dot blot, qPCR, RT-PCR, bioplexing or by means of biosensors and/or
wherein said detection is carried out by enzymatic, fluorescence, immunoenzymatic, immunofluorescence, chemiluminescence, electrochemical, piezoelectric, magnetic, photoacoustic, photothermal, thermal, radioactive or optical methods.

7. The method according to claim 6 wherein, when said detection is carried out by an electrochemical method, said electrochemical method is selected from potentiometry, voltammetry and impedance and said optical method is selected from colorimetry, LSPR, SPR, SERS, RAMAN, OLED and optical fibre.

8. The method according to any one of claims 4 to 7, wherein, when said probe is modified by the binding of a DNA base with a chemical reporter group, with an enzyme, with a fluorophore or with a thiol group SH or with an amino group NH₂, said chemical reporter group is biotin or digoxigenin, said enzyme is alkaline phosphatase, peroxidase glucosidase or ß-galactosidase, and said fluorophore is Cascade Blue (410), Pacific Blue (455), Oregon Green (488X), Bodipy FL-X (510), Fluorescene (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614) Bodipy TR-X (617), Light Cycler 640 (640), Bodipy 630/650-x (650), Cy5 (667) or Cy5.5 (694).

9. The method according to any one of claims 4 to 8, wherein said sample is a food sample, an environmental sample, or a biological sample.

10. The method according to claim 9, wherein said food sample is selected from milk, dairy products and fresh meat from poultry, chicken, turkey, pork and beef; wherein said environmental sample is selected from water or sewage, such as irrigation water, water or sewage taken from animal farms, or from samples taken from breeding environments, premises dedicated to food processing or machinery for processing meat or other foods; said biological sample is selected from animal meat, from human or animal faeces and blood, such as cattle, sheep, swans, pets, or human rectal swabs.

11. An electrode for a biosensor on which oligonucleotide probes as defined in claims 1 or 2 are immobilized.

12. A biosensor comprising an electrode as defined in claim 11.

13. A kit comprising one or more aliquots of an oligonucleotide probe as defined in claims 1 or

14. The kit according to claim 13, wherein said chemical reporter group is biotin or digoxigenin,
said enzyme is alkaline phosphatase, peroxidase glucosidase or ß-galactosidase, and said fluorophore is Cascade Blue (410), Pacific Blue (455), Oregon Green (488X), Bodipy FL-X (510), Fluorescene (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614) Bodipy TR-X (617), Light Cycler 640 (640), Bodipy 630/650-x (650), Cy5 (667) or Cy5.5 (694).

15. Use of the probe as defined in any one of claims 1 to 3, or of the electrode as defined in claim 11, or of the biosensor as defined in claim 12, or of the kit as defined in any one claim 13 or 14 for detecting *C. jejuni, C. coli, C. lari* and *C. upsaliensis* in a sample.

## Patentansprüche

1. Nukleotidsonde von SEQ ID NO 1.

2. Nukleotidsonde nach Anspruch 1, wobei die Sonde mit einer chemischen Reportergruppe, mit einem Enzym, mit einem Fluorophor oder mit einer chemischen Gruppe wie einem Thiol (-SH) oder mit einer Aminogruppe (-NH₂) markiert ist.

3. Nukleotidsonde nach Anspruch 2, wobei die chemische Reportergruppe Biotin oder Digoxigenin ist, das Enzym alkalische Phosphatase, Peroxidase, Glucosidase oder ß-Galactosidase ist und das Fluorophor Cascade Blue (410), Pacific Blue (455), Oregon Green (488X), Bodipy FL-X (510), Fluorescein (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614), Bodipy TR-X (617), Light Cycler 640 (640), Bodipy 630/650-x (650), Cy5 (667) oder Cy5.5 (694) ist.

4. Verfahren zum Detektieren von einem oder mehreren von *C. jejuni, C. coli, C. lari* und *C. upsaliensis* in einer Probe, umfassend
einen Schritt eines Extrahierens von DNA aus der Probe
einen Schritt eines Hybridisierens der aus der Probe extrahierten DNA mit der Nukleotidsonde, wie in einem der Ansprüche 1 bis 3 definiert, und
einen Schritt des Detektierens der Hybridisierung der Sonde an die DNA, wobei die Detektion einer Hybridisierung der Sonde an die DNA das Vorhandensein von mindestens einem von *C. jejuni, C. coli, C. lari* und *C. upsaliensis* in der Probe anzeigt.

5. Verfahren nach Anspruch 4, wobei dem DNA-Extraktionsschritt eines oder mehrere der Folgenden vorangehen
ein Schritt einer bakteriellen Anreicherung in der Probe, und/oder
ein Schritt einer Konzentration von Bakterienzellen durch eine Zentrifugation und/oder eine Filterung,

6. Verfahren nach Anspruch 4 oder 5, wobei die DNA vor dem Hybridisierungsschritt mit RNAse behandelt wird, und/oder
wobei die Hybridisierungs- und Detektionsschritte durch PCR, Blotting, Microarray, FISH, Southern-Blot, Dot-Blot, qPCR, RT-PCR, Bioplexing oder mittels Biosensoren ausgeführt werden und/oder
wobei die Detektion durch enzymatische, Fluoreszenz-, immunenzymatische, Immunfluoreszenz-, Chemilumineszenz-, elektrochemische, piezoelektrische, magnetische, photoakustische, photothermische, thermische, radioaktive oder optische Verfahren ausgeführt wird.

7. Verfahren nach Anspruch 6, wobei, wenn die Detektion durch ein elektrochemisches Verfahren ausgeführt wird, das elektrochemische Verfahren ausgewählt ist aus Potentiometrie, Voltammetrie und Impedanz und das optische Verfahren ausgewählt ist aus Kolorimetrie, LSPR, SPR, SERS, RAMAN, OLED und optischer Faser.

8. Verfahren nach einem der Ansprüche 4 bis 7, wobei, wenn die Sonde durch das Binden einer DNA-Base mit einer chemischen Reportergruppe, mit einem Enzym, mit einem Fluorophor oder mit einer Thiolgruppe SH oder mit einer Aminogruppe NH₂ modifiziert ist, die chemische Reportergruppe Biotin oder Digoxigenin ist, das Enzym alkalische Phosphatase, Peroxidase, Glucosidase oder ß-Galactosidase ist, und das Fluorophor Cascade Blue (410), Pacific Blue (455), Oregon Green (488X), Bodipy FL-X (510), Fluorescein (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614), Bodipy TR-X (617), Light Cycler 640 (640), Bodipy 630/650-x (650), Cy5 (667) oder Cy5.5 (694) ist.

9. Verfahren nach einem der Ansprüche 4 bis 8, wobei die Probe eine Lebensmittelprobe, eine Umgebungsprobe oder eine biologische Probe ist.

10. Verfahren nach Anspruch 9, wobei die Lebensmittelprobe ausgewählt ist aus Milch, Milchprodukten und frischem Fleisch von Geflügel, Huhn, Pute, Schwein und Rind; wobei die Umgebungsprobe ausgewählt ist aus Wasser oder Abwasser, wie Bewässerungswasser, aus Tierhaltungsbetrieben entnommenem Wasser oder Abwasser, oder aus Proben, die aus Zuchtumgebungen, für die Lebensmittelverarbeitung bestimmten Räumlichkeiten oder Maschinen zum Verarbeiten von Fleisch oder anderen Lebensmitteln entnommen wurden; die biologische Probe ausgewählt ist aus tierischem Fleisch, aus menschlichen oder tierischen Fäkalien und Blut, wie Rindern, Schafen, Schwänen, Haustieren, oder menschlichen Rektalabstrichen.

11. Elektrode für einen Biosensor, auf der Oligonukleotidsonden immobilisiert sind, wie in den Ansprüchen 1 oder 2 definiert.

12. Biosensor, umfassend eine Elektrode, wie in Anspruch 11 definiert.

13. Kit, umfassend ein oder mehrere Aliquoten einer Oligonukleotidsonde, wie in Anspruch 1 oder 2 definiert.

14. Kit nach Anspruch 13,
wobei die chemische Reportergruppe Biotin oder Digoxigenin ist, das Enzym alkalische Phosphatase, Peroxidase, Glucosidase oder β-Galactosidase ist und das Fluorophor Cascade Blue (410), Pacific Blue (455), Oregon Green (488X), Bodipy FL-X (510), Fluorescein (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614), Bodipy TR-X (617), Light Cycler 640 (640), Bodipy 630/650-x (650), Cy5 (667) oder Cy5.5 (694) ist.

15. Verwendung der Sonde wie in einem der Ansprüche 1 bis 3 definiert oder der Elektrode wie in Anspruch 11 definiert oder des Biosensors wie in Anspruch 12 definiert oder des Kits wie in Anspruch 13 oder 14 definiert, zum Detektieren von *C. jejuni, C. coli, C. lari* und *C. upsaliensis* in einer Probe.

## Revendications

1. Sonde nucléotidique de SEQ ID NO 1.

2. Sonde nucléotidique selon la revendication 1, dans laquelle ladite sonde est marquée avec un groupe rapporteur chimique, avec une enzyme, avec un fluorophore ou avec un groupe chimique tel qu'un thiol (-SH) ou avec un groupe amino (-NH₂).

3. Sonde nucléotidique selon la revendication 2, dans laquelle ledit groupe rapporteur chimique est biotine ou digoxigénine, ladite enzyme est phosphatase alcaline, peroxydase glucosidase ou ß-galactosidase, et ledit fluorophore est Cascade Blue (410), Pacific Blue (455), Oregon Green (488X), Bodipy FL-X (510), Fluorescene (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614) Bodipy TR-X (617), Light Cycler 640 (640), Bodipy 630/650-x (650), Cy5 (667) ou Cy5.5 (694).

4. Procédé permettant de détecter un ou plusieurs parmi *C. jejuni, C. coli, C. lari* et *C. upsaliensis* dans un échantillon comprenant
une étape consistant à extraire de l'ADN dudit échantillon
une étape consistant à hybrider l'ADN extrait dudit échantillon avec la sonde nucléotidique selon l'une quelconque des revendications 1 à 3 et
une étape consistant à détecter l'hybridation de ladite sonde audit ADN, dans laquelle la détection d'une hybridation de ladite sonde audit ADN indique la présence d'au moins l'un parmi *C. jejuni, C. coli, C. lari* et *C. upsaliensis* dans ledit échantillon.

5. Procédé selon la revendication 4 dans lequel ladite étape d'extraction d'ADN est précédée d'une ou plusieurs de ce qui suit
une étape d'enrichissement bactérien dans ledit échantillon, et/ou
une étape de concentration de cellules bactériennes par centrifugation et/ou filtration,

6. Procédé selon la revendication 4 ou 5, dans lequel ledit ADN est traité avec de l'ARNase avant ladite étape d'hybridation, et/ou
dans lequel lesdites étapes d'hybridation et de détection sont effectuées par PCR, buvardage, micromatrice, FISH, buvardage de Southern, hybridation sur tache, qPCR, RT-PCR, multiplexage biologique ou au moyen de biocapteurs et/ou
dans lequel ladite détection est effectuée par des procédés enzymatiques, de fluorescence, immunoenzymatiques, d'immunofluorescence, de chimiluminescence, électrochimiques, piézoélectriques, magnétiques, photoacoustiques, photothermiques, thermiques, radioactifs ou optiques.

7. Procédé selon la revendication 6 dans lequel, lorsque ladite détection est effectuée par un procédé électrochimique, ledit procédé électrochimique est choisi parmi potentiométrie, voltamétrie et impédance et ledit procédé optique est choisi parmi colorimétrie, LSPR, SPR, SERS, RAMAN, OLED et fibre optique.

8. Procédé selon l'une quelconque des revendications 4 à 7, dans lequel, lorsque ladite sonde est modifiée par la liaison d'une base d'ADN avec un groupe rapporteur chimique, avec une enzyme, avec un fluorophore ou avec un groupe thiol SH ou avec un groupe amino NH₂, ledit groupe rapporteur chimique est biotine ou digoxigénine, ladite enzyme est phosphatase alcaline, peroxydase glucosidase ou ß-galactosidase, et ledit fluorophore est Cascade Blue (410), Pacific Blue (455), Oregon Green (488X), Bodipy FL-X (510), Fluorescene (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614) Bodipy TR-X (617), Light Cycler 640 (640), Bodipy 630/650-x (650), Cy5 (667) ou Cy5.5 (694).

9. Procédé selon l'une quelconque des revendications 4 à 8, dans lequel ledit échantillon est un échantillon alimentaire, un échantillon environnemental ou un échantillon biologique.

10. Procédé selon la revendication 9, dans lequel ledit échantillon alimentaire est choisi parmi lait, produits laitiers et viande fraîche provenant de volaille, de poulet, de dinde, de porc et de bœuf ; dans lequel ledit échantillon environnemental est choisi parmi eau ou eaux usées, telles qu'eau d'irrigation, eau ou aux usées prélevées dans des fermes animalières, ou parmi des échantillons prélevés dans des environnements d'élevage, des locaux dédiés à la transformation alimentaire ou des machines permettant de traiter de la viande ou d'autres aliments ; ledit échantillon biologique est choisi parmi de la viande animale, parmi des matières fécales et du sang humains ou animaux, tels que bovins, de moutons, cygnes, animaux de compagnie ou prélèvements rectaux humains.

11. Électrode destinée à un biocapteur sur lequel des sondes d'oligonucléotide selon la revendication 1 ou 2 sont immobilisées.

12. Biocapteur comprenant une électrode selon la revendication 11.

13. Trousse comprenant une ou plusieurs aliquotes d'une sonde oligonucléotidique selon la revendication 1 ou 2.

14. Trousse selon la revendication 13,
dans laquelle ledit groupe rapporteur chimique est biotine ou digoxigénine, ladite enzyme est phosphatase alcaline, peroxydase glucosidase ou ß-galactosidase, et ledit fluorophore est Cascade Blue (410), Pacific Blue (455), Oregon Green (488X), Bodipy FL-X (510), Fluorescene (520), 5,6FAM (518), Oregon Green (524), TET (536), Bodipy R6G-X (547), JOE (548), HEX (556), Cy3 (570), Rhodamine Red-X (580), TAMARA (580), Cy3.5 (596), ROX (605), Texas Red-X (614) Bodipy TR-X (617), Light Cycler 640 (640), Bodipy 630/650-x (650), Cy5 (667) ou Cy5.5 (694).

15. Utilisation de la sonde selon l'une quelconque des revendications 1 à 3, ou de l'électrode selon la revendication 11, ou du biocapteur selon la revendication 12, ou de la trousse selon l'une quelconque parmi la revendication 13 ou 14 pour la détection de *C*. *jejuni, C. coli, C. lari* et *C*. *upsaliensis* dans un échantillon.
